# EUROPÄISCHE PATENTSCHRIFT

(11) **EP 0 545 304 B1**
(45) Veröffentlichungstag und Bekanntmachung des Hinweises auf die Patenterteilung: **19.06.2002**
(21) Anmeldenummer: 92120289.1
(22) Anmeldetag: 27.11.1992
(51) Int. Cl.: C07F 17/00, C07F 7/08, C07C 1/207, C08F 4/00, C08F 4/602, C07C 45/46, C07C 13/465, C07C 13/547, C07C 13/66

(54) **Verfahren zur Herstellung substituierter Indene und ihre Verwendung als Ligandsysteme für Metallocen-Katalysatoren**
Process for the preparation of substituted indenes and their use as ligands for metallocene catalysts
Procédé de préparation d'indenes substitués et leur application comme ligandes de catalyseurs métallocènes

(30) Priorität: 30.11.1991 DE 4139594
(43) Veröffentlichungstag der Anmeldung: 09.06.1993
(73) Patentinhaber: Basell Polyolefine GmbH, 67056 Ludwigshafen (DE)
(72) Erfinder: Rohrmann, Jürgen Dr., 6233 Kelkheim/Ts. (DE); Kuber, Frank Dr., 6370 Oberursel (DE)

(56) Entgegenhaltungen:
- EP-A- 0 320 762
- EP-A- 0 344 887
- EP-A- 0 366 290
- EP-A- 0 407 870
- EP-A- 0 485 821
- EP-A- 0 485 823
- EP-A- 0 518 092
- EP-A- 0 519 236
- EP-A- 0 519 237
- EP-A- 0 529 908
- AT-B- 344 153
- JP-A- 2 208 213
- CHEMICAL ABSTRACTS, vol. 90, 1979, Columbus, Ohio, US; abstract no. 103691p, FABRYCY, A. ET AL. Seite 567 ;
- BULLETIN DE LA SOCI T CHIMIQUE DE FRANCE Nr. 11, 1973, Seiten 3092 - 3095 OLIVIER, M. ET AL.
- JOURNAL OF THE AMERICAN CHEMICAL SOCIETY Bd. 72, 1950, Seiten 3286 - 3287 HART, R.T. ET AL.
- THE JOURNAL OF ORGANIC CHEMISTRY Bd. 46, 1981, Seiten 3758 - 3760 PINNICK, H.W. ET AL.
- THE JOURNAL OF ORGANIC CHEMISTRY Bd. 43, Nr. 16, 1978, Seiten 3126 - 3131 PINES, S.H. ET AL.
- DOYAMA K. ET AL. CHEMISTRY LETTERS 1988, Seiten 901 - 904
- BAUM A.A JOURNAL OF THE AMERICAN CHEMICAL SOCIETY Bd. 94, Nr. 19, 1972, Seiten 6866 - 6867
- KUCK D. ET AL. CHEMISCAHE BERICHTE 1987, Seiten 589 - 595
- FUKUOKA M. ET AL. CHEMICAL PHARMACEUTICAL BULLETIN Bd. 31, Nr. 9, 1983, Seiten 3113 - 3128
- NEUDECK H.K. MONATSHEFTE FOR CHEMIE Bd. 118, 1987, Seiten 627 - 657
- NEUDECK H.K. MONATSHEFTE FOR CHEMIE Bd. 120, 1989, Seiten 597 - 621

## Beschreibung

Die vorliegende Erfindung betrifft ein einfaches Verfahren zur Herstellung von am Fünf- und am Sechsring substituierter Indenderivate.

Verbindungen dieses Typs sind wichtige Zwischenprodukte bei der Herstellung von Metallocen-Komplexen. Insbesondere die entsprechenden verbrückten, chiralen Zirkon-Derivate besitzen als hochaktive Katalysatoren bei der Olefinpolymerisation große Bedeutung (vgl. EP-A-129 368, EP-A-366 290). Durch Variation des Ligandsystems, z. B. durch Substitution, können die Katalysatoreigenschaften gezielt beeinflußt werden. Hierdurch ist es möglich, die Polymerausbeute, die Molmasse, die Taktizität oder den Schmelzpunkt der Polymeren im gewünschten Maß zu verändern (New J. Chem. 14(1990) 499; Organomet. 9 (1990) 3098; Angew. Chem. 102 (1990) 339; EP-A 316 155; EP-A 351 392).

Weiterhin können Indene auch als Monomere bei der Homopolymerisation oder Copolymerisation mit anderen Olefinen eingesetzt werden (vgl. Macromol. 22 (1989) 3824; Bull. Soc. Chim. Fr. 6 (1969) 2039).

Die wenigen in der Literatur beschriebenen substituierten Indene sind jedoch in der Regel nur über vielstufige Synthesen in geringen Ausbeuten zugänglich. Meistens werden sie aus den entsprechend substituierten 1-Indanonen durch Reduktion und anschließende Dehydratisierung erhalten. Die entsprechenden Indanone sind in mehrstufigen Synthesen ausgehend von substituierten Aromaten erhältlich (Bull. Soc. Chim. Fr. 6 (1969) 1981; Acta Chem. Scand. B 30 (1976) 527; Austr. J. Chem. 29 (1970) 2572; Chem. Lett. (1981) 729; Ber. 97(12) (1964) 3461).

Darüberhinaus sind bestimmte Substitutionsmuster auf diesem Wege nicht zugänglich.

EP-A 0 518 092, EP-A 0 519 236 und EP-A 0 519 237 offenbaren Dimethylsilandiyl bis (-2-methylbenzindenyl)-zirkoniumdichlorid, jedoch nicht als entsprechende in den oder dessen Vorstufe, sowie Synthesewege dorthin.

Es bestand somit die Aufgabe, ein Verfahren zur Herstellung der obengenannten Indene zu finden, das die aus dem Stand der Technik bekannten Nachteile vermeidet. Solche Indene ermöglichen den Zugang zu neuen Metallocenkomplexen.

Es wurde gefunden, daß Aromaten der nachstehenden Formel I mit Derivaten der Propionsäure, die α-ständig eine Abgangsgruppe tragen, und einem Friedel-Crafts-Katalysator in hohen Ausbeuten zu substituierten 1-Indanonen reagieren. Dieses Ergebnis war völlig unerwartet, da diese Produkte nur mit Derivaten der Propionsäure zu erwarten gewesen wären, die in β-Stellung eine Abgangsgruppe tragen (vgl. J. Amer. Chem. Soc. 72 (1950) 3286).

Darüberhinaus ist diese Synthese ein technisch einfach zu handhabender Einstufenprozeß. Die Indanone können anschließend nach bekannten Methoden in die entsprechenden Indene überführt werden. Gleichzeitig ermöglicht das erfindungsgemäße Verfahren die Herstellung von neuen Verbindungen des genannten Strukturtyps.

Die vorliegende Erfindung betrifft daher ein Verfahren zur Herstellung einer am Fünfring und am Sechsring substituierten Verbindung der Formel IV oder deren Isomer der Formel IVa 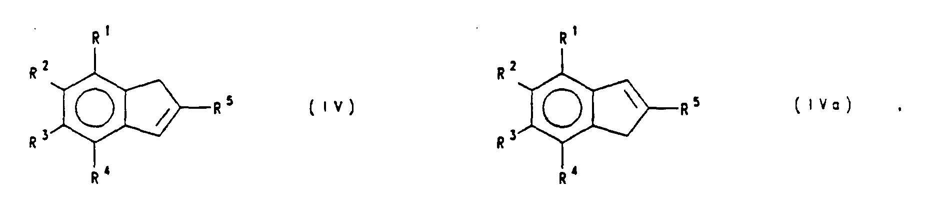 worin
R¹, R², R³, R⁴ und R⁵ gleich oder verschieden sind und Wasserstoff, (C₁-C₂₀)Alkyl, (C₆-C₁₄)Aryl, (C₁-C₁₀)Alkoxy, (C₂-C₁₀)Alkenyl, (C₇-C₂₀)Arylalkyl, (C₇-C₂₀)Alkylaryl, (C₆-C₁₀)Aryloxy, (C₁-C₁₀)Fluoralkyl, (C₆-C₁₀)Halogenaryl, (C₂-C₁₀)Alkinyl, einen Rest - SiR⁶₃, wobei R⁶ für (C₁-C₁₀)Alkyl steht, ein Halogenatom oder einen heteroaromatischen Rest mit 5 oder 6 Ringgliedern, der ein oder mehrere Heteroatome enthalten kann, bedeuten, oder benachbarte Reste R¹-R⁴ bilden mit den sie verbindenden Atomen einen oder mehrere Ringe, dadurch gekennzeichnet, daß man eine Verbindung der Formel I 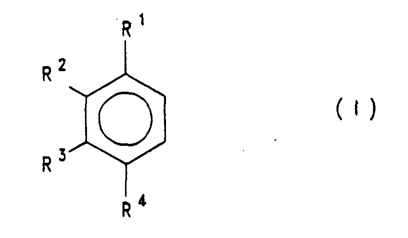 mit einer Verbindung der Formel II 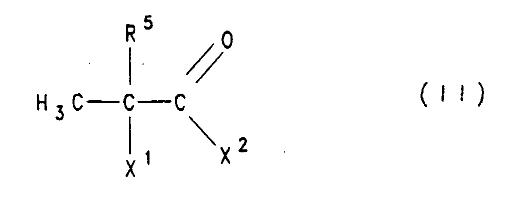 oder deren Anhydrid in Gegenwart eines Friedel-Crafts-Katalysators zu einer Verbindung der Formel III bzw. der Formel IIIa umsetzt, 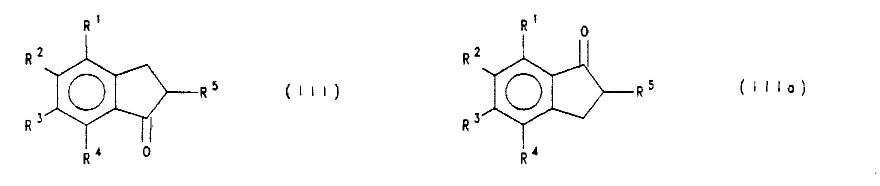 wobei R¹-R⁵ die genannten Bedeutungen besitzen und X¹ und X² gleich oder verschieden sind und für eine nucleophile Abgangsgruppe stehen, und diese nach bekannten Methoden durch Reduktion und Dehydratisierung in die Verbindung der Formel IV oder IV a überführt.

Dabei steht Alkyl für geradkettiges oder verzweigtes Alkyl. Halogen bedeutet Fluor, Chlor, Brom oder Jod, insbesondere Fluor oder Chlor. Beispiele für heteroaromatische Reste sind Thienyl, Furyl oder Pyridyl.

Die Indanone können in Abhängigkeit des Substitutionsmusters am Aromaten in Form zweier Konstitutionsisomere der Formel III bzw. IIIa anfallen. Diese können in reiner Form oder als Gemisch nach literaturbekannten Methoden mit Reduktionsmitteln wie NaBH₄ oder LiAlH₄ zu den entsprechenden Indanolen reduziert und anschließend mit Säuren wie Schwefelsäure, Oxalsäure, p-Toluolsulfonsäure oder auch durch Behandlung mit wasserentziehenden Substanzen wie Magnesiumsulfat, Natriumsulfat, Aluminiumoxid, Kieselgel oder Molekularsiebe zu Indenen der Formel IV bzw. IVa dehydratisiert werden (Bull. Soc. Chim. Fr. 11 (1973) 3092; Organomet. 9 (1990) 3098 und die Ausführungsbeispiele).

X¹ und X² bedeuten bevorzugt ein Halogenatom, eine Hydroxylgruppe, eine Tosylgruppe oder eine (C₁-C₁₀)Alkoxygruppe; insbesondere ein Halogenatom, besonders bevorzugt Brom oder Chlor.

Geeignete Friedel-Crafts-Katalysatoren sind z. B. AlCl₃, AlBr₃, FeCl₃, SbCl₅, SnCl₄, BF₃, TiCl₄, ZnCl₂, HF, H₂SO₄, Polyphosphorsäure, H₃PO₄ oder eine AlCl₃/NaCl-Schmelze; insbesondere AlCl₃.

In den Formeln IV bzw. IVa gilt bevorzugt, daß
R¹, R², R³, R⁴ gleich oder verschieden sind und Wasserstoff, (C₁-C₁₀)Alkyl, (C₆-C₁₄)Aryl, (C₁-C₄)Alkoxy, (C₂-C₆)Alkenyl, (C₁-C₆)Fluoralkyl, ein Halogenatom oder einen heteroaromatischen Rest mit 5 oder 6 Ringgliedern, der ein oder mehrere Heteroatome enthalten kann, bedeuten, oder benachbarte Reste R¹-R⁴ bilden mit den sie verbindenden Atomen einen Ring, und R⁵ (C₁-C₁₀)Alkyl bedeutet.
Insbesondere gilt, daß
R¹, R², R³, R⁴ gleich oder verschieden sind und Wasserstoff oder (C₁-C₁₀)Alkyl bedeuten, oder die Reste R¹ und R² oder R³ und R⁴ bilden mit den sie verbindenden Atomen einen Ring, und R⁵ für Methyl steht.

Die Ausgangsverbindungen der Formeln I und II sind bekannt und im Handel erhältlich, oder sie lassen sich nach literaturbekannten Verfahren herstellen.

Die Umsetzung wird in einem inerten Lösemittel durchgeführt. Bevorzugt wird Methylenchlorid oder CS₂ eingesetzt. Sind die Ausgangskomponenten flüssig, kann auch auf ein Lösemittel verzichtet werden.

Die Molverhältnisse der Ausgangsverbindungen einschließlich des Friedel-Crafts-Katalysators können innerhalb weiter Grenzen schwanken. Bevorzugt ist das Molverhältnis von Verbindung I : II : Katalysator = 1 : 0,5-1,5 : 1-5; insbesondere 1 : 1 : 2,5-3.

Die Reaktionstemperatur beträgt bevorzugt 0°C bis 130°C, insbesondere 25°C bis 80°C.

Die Reaktionszeiten schwanken in der Regel zwischen 30 min und 100 h, vorzugsweise zwischen 2 h und 30 h.

Bevorzugt wird eine Mischung der Verbindungen I und II vorgelegt und der Friedel-Crafts-Katalysator zudosiert. Auch die umgekehrte Zugabefolge ist möglich.

Die Indanone der Formel III bzw. IIIa können durch Destillation, Säulenchromatographie oder Kristallisation gereinigt werden.

Die substituierten Indene können als Doppelbindungsisomere anfallen (IV/IVa). Diese können von Nebenprodukten durch Destillation, Säulenchromatographie oder Kristallisation gereinigt werden.

Das erfindungsgemäße Verfahren zeichnet sich besonders dadurch aus, daß unterschiedlich substituierte Indene in einer einfachen und kurzen Synthese in hoher Ausbeute erhalten werden können. Das Substitutionsmuster am Fünf- und Sechsring kann dabei in einem sehr weiten Bereich variiert werden. Dadurch sind auch neue Indenderivate zugänglich.

Gegenstand der vorliegenden Erfindung ist ferner die Verwendung der Indenderivate IV/IVa als Zwischenprodukt bei der Herstellung von Metallocenkomplexen, insbesondere von solchen der nachstehenden Formel VI. Verbindung der Formel VI mit am Fünfring und am Sechsring substituierten Indenylliganden worin p ist 0, oder Verbindung der Formel VI mit am Fünfring und am Sechsring substituierten Indenylliganden, die in 2,6-, 2,4,6-. 2,4,5,6- oder 2,4,5,6,7-Stellung substituiert sind, worin p ist 1, 2 oder 3 : 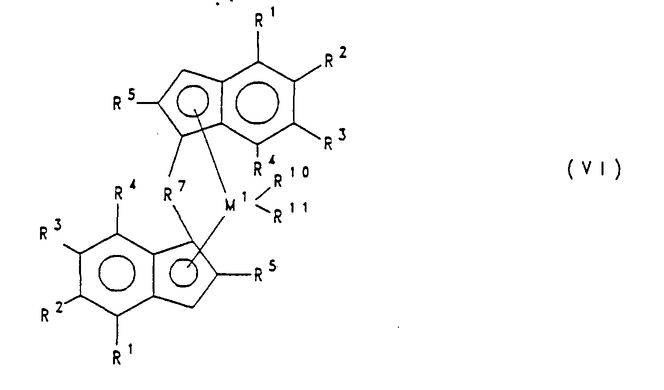 M¹ Titan, Zirkon, Hafnium, Vanadium, Niob oder Tantal ist,
R¹, R², R³, R⁴ und R⁵ gleich oder verschieden sind und
Wasserstoff, (C₁-C₂₀)Alkyl, (C₆-C₁₄)Aryl, (C₁-C₁₀)Alkoxy, (C₂-C₁₀)Alkenyl, (C₇-C₂₀)Arylalkyl, (C₇-C₂₀)Alkylaryl, (C₆-C₁₀)Aryloxy, (C₁-C₁₀)Fluoralkyl, (C₆-C₁₀)Halogenaryl, (C₂-C₁₀)Alkinyl, einen Rest -SiR⁶₃, wobei R⁶ für (C₁-C₁₀)Alkylsteht, ein Halogenatom oder einen heteroaromatischen Rest mit 5 oder 6 Ringgliedern, der ein oder mehrere Heteroatome enthalten kann, bedeuten, oder benachbarte Reste R¹-R⁴ bilden mit den sie verbindenden Atomen einen oder mehrere Ringe,
R⁷ ein Rest 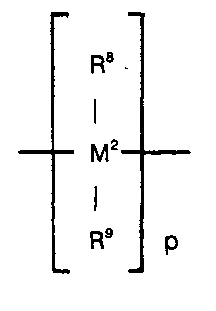 ist, wobei
M² Kohlenstoff, Silizium, Germanium oder Zinn bedeutet,
R⁸ und R⁹ gleich oder verschieden sind und Wasserstoff, (C₁-C₂₀)Alkyl, (C₆-C₁₄)Aryl, (C₁-C₁₀)Alkoxy, (C₂-C₁₀)Alkenyl, (C₇-C₂₀)Arylalkyl, (C₇-C₂₀)Alkylaryl, (C₆-C₁₀)Aryloxy, (C₁-C₁₀)Fluoralkyl, (C₆-C₁₀)Halogenaryl, (C₂-C₁₀)Alkinyl oder Halogen bedeuten, oder R⁸ und R⁹ zusammen mit dem sie verbindenden Atom einen Ring bilden, und
R¹⁰ und R¹¹ gleich oder verschieden sind und Wasserstoff, (C₁-C₁₀)Alkyl, (C₁-C₁₀)Alkoxy, (C₆-C₁₀)Aryl, (C₆-C₁₀)Aryloxy, (C₂-C₁₀)Alkenyl, (C₇-C₄₀)Arylalkyl, (C₇-C₄₀)Alkylaryl, (C₈-C₄₀)Arylalkenyl, Hydroxy oder ein Halogenatom bedeuten.

Bevorzugt gilt, daß
M¹ Zirkon oder Hafnium, insbesondere Zirkon, ist,
R¹, R², R³, R⁴ gleich oder verschieden sind und Wasserstoff, (C₁-C₁₀)Alkyl, (C₆-C₁₄)Aryl, (C₁-C₄)Alkoxy, (C₂-C₆)Alkenyl, (C₁-C₆)Fluoralkyl, ein Halogenatom oder einen heteroaromatischen Rest mit 5 oder 6 Ringgliedern, der ein oder mehrere Heteroatome enthalten kann, bedeuten und R⁵ (C₁-C₁₀)Alkyl bedeutet, oder benachbarte Reste R¹-R⁴ bilden mit den sie verbindenden Atomen einen Ring,
M² Kohlenstoff oder Silizium, insbesondere Silizium, bedeutet, R⁸ und R⁹ gleich oder verschieden sind und Wasserstoff, (C₁-C₆)Alkyl, (C₆-C₁₀)Aryl, (C₁-C₆)Alkoxy, (C₂-C₄)Alkenyl, (C₇-C₁₀)Arylalkyl, (C₇-C₁₀)Alkylaryl bedeuten oder R⁸ und R⁹ zusammen mit dem sie verbindenden Atom einen Ring bilden, p 1 oder 2,
bevorzugt 1, ist, und R¹⁰ und R¹¹ gleich oder verschieden sind und Wasserstoff, (C₁-C₃)Alkyl, insbesondere Methyl, (C₁-C₃)Alkoxy, (C₆-C₈)Aryl, (C₆-C₈)Aryloxy, (C₂-C₄)Alkenyl, (C₇-C₁₀)Arylalkyl, (C₇-C₁₀)Alkylaryl, (C₈-C₁₂)Arylalkenyl oder ein Halogenatom, bevorzugt Chlor, bedeuten.

Vorzugsweise sind die Reste R¹⁰ und R¹¹ gleich und bedeuten Chlor oder Methyl. M² steht insbesondere für Silizium und die Reste R⁸, R⁹ sind gleich oder verschieden und bedeuten vorzugsweise (C₁-C₆)Alkyl, bevorzugt Methyl, oder (C₆-C₁₀)Aryl.

Weiterhin gilt für die Verbindungen der Formel VI, daß bevorzugt R⁵ und R³; R¹, R³ und R⁵; R¹, R², R³ und R⁵ oder alle Reste R¹-R⁵ ungleich Wasserstoff sind und vorzugsweise für (C₁-C₄)Alkyl stehen. Besonders bevorzugt sind die Reste R¹, R³ und R⁵ ungleich Wasserstoff, sind gleich oder verschieden und bedeuten (C₁-C₄) Alkyl.

Die bevorzugten Substitutionsmuster an den Indenylresten sind somit 2,6-, 2,4,6-, 2,4,5-, 2,4,5,6- und 2,4,5,6,7-, insbesondere 2,4,6- und 2,4,5- für p = 0 in (VI) und 2,6-, 2,4,6-, 2,4,5,6- und 2,4,5,6,7-, insbesondere 2,4,6- für p = 1,2,3 in (VI). Dabei ist die 2-Stellung an den Indenylresten (R⁵) vorzugsweise durch eine Methylgruppe substituiert. Weiterhin gilt für die Verbindungen der Formel VI, mit p = 0 daß die Indenylreste benzoannelliert sind.

Von besonderer Bedeutung sind die in den Ausführungsbeispielen genannten Verbindungen VI.

Ausgehend von den Indenen der Formeln IV und IVa, die als Isomerengemisch eingesetzt werden können, verläuft die Herstellung der Metallocene VI nach literaturbekannten Verfahren (vgl. AU-A-31478/89, J. Organomet. Chem. 342 (1988) 21, EP-A 284 707 und die Ausführungsbeispiele) entsprechend dem folgenden Reaktionsschema:

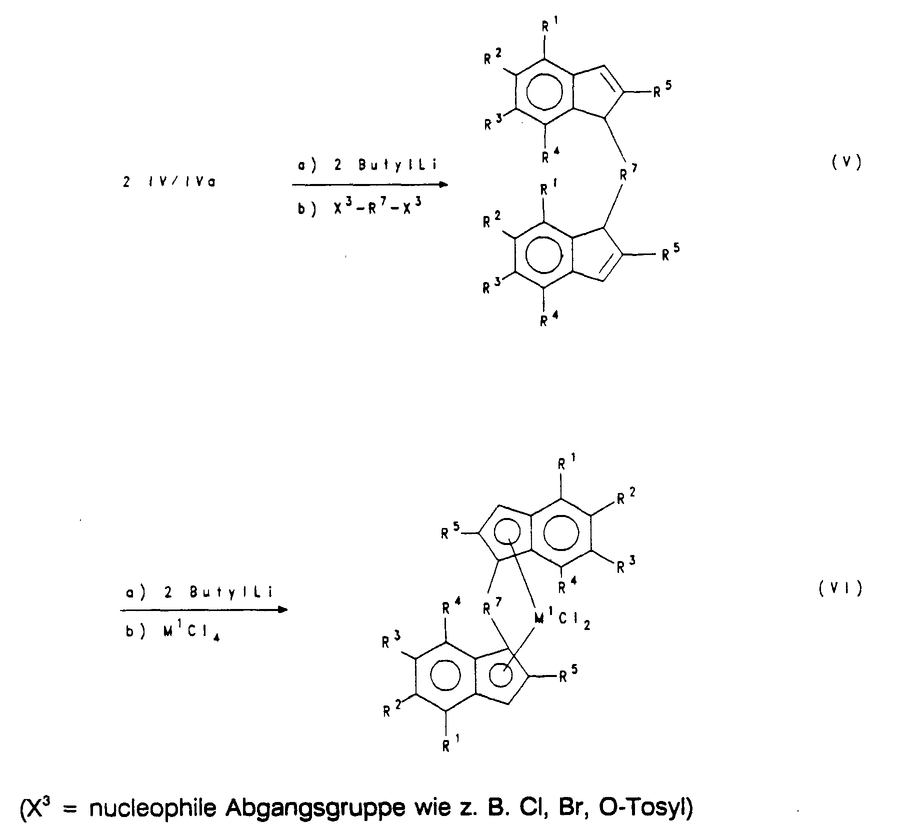

Die Metallocenhalogenide der Formel VI können nach literaturbekannten Methoden, z. B. durch Umsetzungen mit Alkylierungsmitteln, wie Lithiumalkylen, zu den entsprechenden Mono- oder Dialkylverbindungen derivatisiert werden (J. Amer. Chem. Soc. 95 (1973) 6263).

Die verbrückten Ligandsysteme der Formel V können in Abhängigkeit vom Substitutionsmuster des Indens als Konstitutionsisomere anfallen. Werden diese nicht getrennt, so entstehen Konstitutionsisomere Metallocene der Formel VI. Die Metallocene der Formel VI fallen als Gemisch der racemischen mit der meso-Form an. Die Trennung der isomeren Formen, insbesondere die Abtrennung der für die Olefin-Polymerisation unerwünschten meso-Form, ist im Prinzip bekannt (AU-A-31478/89; J. Organomet. Chem. 342 (1988) 21; EP-A 284 707). Sie erfolgt in der Regel durch Extraktion oder Umkristallisation mit verschiedenen Lösemitteln.

Gegenstand der vorliegenden Erfindung ist weiterhin die Verwendung der Verbindungen der Formel VI als Katalysatorkomponente bei der Olefinpolymerisation.

Die Metallocene VI sind hochaktive Katalysatoren und z. B. für die Herstellung von Olefinpolymeren mit hoher Isotaktizität und hoher Molmasse geeignet.

Die Polymerisation oder Copolymerisation wird in bekannter Weise in Lösung, in Suspension oder in der Gasphase, kontinuierlich oder diskontinuierlich, ein- oder mehrstufig bei einer Temperatur von 0 bis 150°C, vorzugsweise 30 bis 80°C, durchgeführt. Polymerisiert oder copolymerisiert werden Olefine der Formel R^{a}-CH=CH-R^{b}. In dieser Formel sind R^{a} und R^{b} gleich oder verschieden und bedeuten ein Waserstoffatom oder einen Alkylrest mit 1 bis 14 C-Atomen. R^{a} und R^{b} können jedoch auch mit den sie verbindenden C-Atomen einen Ring bilden. Beispiele für solche Olefine sind Ethylen, Propylen, 1-Buten, 1-Hexen, 4-Methyl-1-penten, 1-Octen, Norbornen Dimethanooctahydronaphtalin oder Norbornadien. Insbesondere werden Propylen und Ethylen polymerisiert (vgl. z. B. EP-A 129 368).

Als Cokatalyatoren werden bevorzugt Aluminoxane verwendet (vgl. EP-A 129 368; Polyhedron 9 (1990)429 und die Ausführungsbeispiele).

Erfindungsgemäß können an Stelle oder neben eines Aluminoxans Verbindungen der Formeln RₓNH₄₋ₓBR'₄, RₓPH₄₋ₓBR'₄, R₃CBR'₄ oder BR'₃ als geeignete Cokatalysatoren verwendet werden. In diesen Formeln bedeutet x eine Zahl von 1 bis 4, bevorzugt 3, die Reste R sind gleich oder verschieden, bevorzugt gleich, und bedeuten C₁-C₁₀-Alkyl, C₆-C₁₈-Aryl oder 2 Reste R bilden zusammen mit dem sie verbindenden Atom einen Ring, und die Reste R' sind gleich oder verschieden, bevorzugt gleich, und stehen für C₆-C₁₈-Aryl, das durch Alkyl, Haloalkyl oder Fluor substituiert sein kann (EP-A 277 003, 277 004, 426 638, 427 697).

Die nachfolgenden Beispiele dienen der näheren Erläuterung der Erfindung.

### Beispiel A

### 2,5,7-Trimethyl-1-indanon (1)

Eine Lösung von 34,4 g (324 mmol) m-Xylol (99 %) und 74 g (324 mmol) 2-Bromisobuttersäurebromid (98 %) in 600 ml Methylenchlorid p. a. wurde bei Raumtemperatur unter kräftigem Rühren über einen Feststoffdosiertrichter langsam mit 107 g (810 mmol) AlCl₃ versetzt, wobei eine heftige Gasentwicklung einsetzte. Die Mischung wurde 15 h bei Raumtemperatur gerührt, auf Eiswasser gegossen, das mit 25 ml konz. HCI angesäuert wurde und mehrmals ausgeethert. Die vereinigten organischen Phasen wurden zunächst mit einer gesättigten NaHCO₃-Lösung und dann mit einer gesättigten NaCl-Lösung gewaschen und mit Magnesiumsulfat getrocknet. Das nach Abziehen des Lösemittels unter vermindertem Druck zurückgebliebene Öl wurde über eine kurze Destillationsbrücke destilliert. Bei 81-90°C/0,1-0,2 mbar gingen 52,4 g des Indanons 1 in Form eines farblosen Öls über, das bei Raumtemperatur kristallisierte. Die Ausbeute betrug 93 %.
¹H-NMR-Spektrum (100 MHz, CDCl₃): 7,05 (1,s), 6,87 (1,s), 3,25 (1,q), 2,43-2,80 (2,m), 2,57 (3,s), 2,35 (3,s), 1,25 (3,d).
Massenspektrum: 174 M⁺, korrektes Zerfallsmuster.

### Beispiel B

### 2,4,6-Trimethylinden (2)

20,4 g (117 mmol) 2,5,7-Trimethyl-1-indanon (1) wurden in 300 ml einer Mischung aus Tetrahydrofuran/Methanol (2:1) gelöst und bei Raumtemperatur mit 6,6 g (175 mmol) NaBH₄ versetzt. Die Mischung wurde noch 1 h gerührt, mit 50 ml halbkonz. HCI versetzt und ausgeethert. Die vereinigten organischen Phasen wurden über Natriumsulfat getrocknet und vom Lösemittel befreit. Der Rückstand wurde in eine Destillationsapparatur überführt und mit 13 g Magnesiumsulfat versetzt. Es wurde ein Vakuum von ca. 10 mbar angelgt und hochgeheizt, bis das Produkt überdestillierte (130-150°C). Die Destillation ergab 17,7 g des Indens 2 als farbloses Öl. Die Ausbeute betrug 96 %.
¹H-NMR-Spektrum (100 MHz, CDCl₃): Doppelbindungsisomere A:B = 2:1
Isomer A: 6,97 (1,s), 6,80 (1,s), 6,50 (1,m), 3,20 (2,m), 2,1-2,3 (9,m).
Isomer B: 6,87 (1,s), 6,70 (1,s), 6,37 (1,m), 3,07 (2,m), 2,1-2,3 (9,m). Massenspektrum: 158 M⁺, korrektes Zerfallsmuster.

### Beispiel C

### 2-Methyl-5,7-diisopropyl-1-indanon (3) bzw. 2-Methyl-4,6-diisopropyl-1-indanon (3a)

Eine Lösung von 84,8 g (523 mmol) 1,3-Diisopropylbenzol und 120 g (523 mmol) 2-Bromisobuttersäurebromid (98 %) in 600 ml Methylenchlorid p. a. wurde bei Raumtemperatur über einen Feststoffdosiertrichter langsam mit 174 g (1300 mmol) AlCl₃ versetzt. Die Mischung wurde noch 20 h zum Rückfluß erhitzt und dann analog Beispiel A aufgearbeitet. Das Rohprodukt wurde an 3 kg Kieselgel 60 chromatographiert. Die Indanone 3 und 3a konnten mit einem Laufmittelgemisch aus Hexan/15 % Essigester separat eluiert werden. Mit dem gleichen Laufmittel folgte in einer weiteren Zone als Nebenprodukt die Verbindung 2-Methyl-5-isopropyl-1-indanon. Eine Trennung der beiden Isomere ist für die weiteren Umsetzungen jedoch nicht erforderlich. Die Gesamtausbeute betrug 93 g (78 %).
¹H-NMR-Spektrum (360 MHz, CDCl₃): Isomerenmischung (3:2) 7,49 (d), 7,36 (d), 7,13 (s), 7,10 (s), 4,15 (sept.), 3,25-3,40 (m), 3,10 (sept.), 2,90-3,00 (m), 2,60-2,73 (m), 1,22-1,30 (m).
Massenspektrum: 230 M⁺, korrektes Zerfallsmuster.

### Beispiel D

### 2-Methyl-4,6-diisopropylinden (4) bzw. 2-Methyl-5,7-diisopropylinden (4a), Variante I

Eine Lösung von 78,5 g (341 mmol) der Isomerenmischung 3/3a in 700 ml eines Lösemittelgemisches aus Tetrahydrofuran/Methanol p.a. (2:1) wurde bei Raumtemperatur mit 19,3 g (511 mmol) NaBH₄ versetzt. Nach 2 h Rühren bei Raumtemperatur wurden 120-130 ml halbkonz. HCI zugesetzt und ausgeethert. Die vereinigten organischen Phasen wurden mit Na₂SO₄ getrocknet. Der nach Abziehen des Lösemittels verbliebene Rückstand wurde in 500 ml Methylenchlorid aufgenommen und mit 6,5 g (34 mmol) p-Toluolsulfonsäure 15 min zum Rückfluß erhitzt. Der nach Abziehen des Lösemittels verbliebene Rückstand wurde an 1,5 kg Kieselgel 60 chromatographiert. Mit einem Laufmittelgemisch aus Hexan/Diisopropylether 20:1 ließen sich 56 g der Isomerenmischung 4/4a in Form eines farblosen Öls isolieren. Die Gesamtausbeute betrug 86 %.
¹H-NMR-Spektrum (100 MHz, CDCl₃): Doppelbindungsisomere (1:1) 7,1 (m), 6,95 (m), 6,60 (m), 6,43 (m), 3,25 (br), 2,75-3,20 (m), 2,12 (d), 1,28 (d), 1,25 (d). Massenspektrum: 214 M⁺, korrektes Zerfallsmuster.

### Beispiel E

### 2-Methyl-4,6-diisopropylinden (4) bzw. 2-Methyl-5,7-diisopropylinden (4a), Variante II

Eine Lösung von 78,5 g (341 mmol) der Isomerenmischung 3/3a in 700 ml eines Lösemittelgemisches aus Tetrahydrofuran/Methanol /Methanol p.a. (2:1) wurde bei Raumtemperatur mit 19,3 g (511 mmol) NaBH₄ versetzt. Nach 2 h Rühren bei Raumtemperatur wurden 120-130 ml halbkonz. HCI zugesetzt und ausgeethert. Die vereinigten organischen Phasen wurden mit Na₂SO₄ getrocknet. Der nach Abziehen des Lösemittels verbliebene Rückstand wurde in eine Destillationsapparatur überführt und mit 50 g Magnesiumsulfat versetzt. Nach Anlegen eines Vakuums von ca. 1 mbar wurde hochgeheizt, bis das Produkt überging (ca. 130°C). Man erhielt 65 g der Isomerenmischung 4/4a als farbloses Öl. Die Ausbeute betrug 90 %.

### Beispiel F

### 2-Methyl-1-indanon (5)

Eine Lösung von 3,91 g (50 mmol) Benzol in 30 ml Methylenchlorid p.a. wurde unter Eiskühlung mit 17,3 g (125 mmol) AlCl₃ versetzt. Anschließend wurden 11,9 g (52 mmol) 2-Bromisobuttersäurebromid zugegeben, 1 h bei 0°C und 2 h bei Raumtemperatur weiter gerührt. Die Mischung wurde noch 15 h unter Rückfluß erhitzt und dann analog Beispiel A aufgearbeitet. Das Rohprodukt wurde an 100 g Kieselgel (Hexan/Methylenchlorid 1:1) chromatographiert. Die Ausbeute betrug 5,1 g (70 %).
¹H-NMR-Spektrum (100 MHz, CDCl₃): 7,5 (m), 3,33 (q), 2,73 (m), 1,30 (d). Massenspektrum: 146 M⁺, korrektes Zerfallsmuster.

### Beispiel G

### 2-Methylinden (6)

Analog Beispiel D wurden 5,0 g (34 mmol) 2-Methyl-1-indanon (5) mit 1,94 g (51 mmol) NaBH₄ reduziert. Der nicht weiter gereinigte Alkohol wurde anschließend in Gegenwart von 0,2 g p-Toluolsulfonsäure in 100 ml Toluol bei 80°C weiter umgesetzt. Nach Chromatographie an 100 g Kieselgel (Hexan/Methylenchlorid 9:1) erhielt man 3,68 g (82 %) 2-Methylinden (6).
¹H-NMR-Spektrum (100 MHz, CDCl₃): 7,2 (4,m), 6,45 (1,m), 3,25 (2,m), 2,1 (3,m).
Massenspektrum: 130 M⁺, korrektes Zerfallsmuster.

### Beispiel H

### 2-Methyl-5-isobutyl-1-indanon (7)

Eine Lösung von 6,71 g (50 mmol) Isobutylbenzol in 30 ml Methylenchlorid p.a. wurde unter Eiskühlung mit 17,3 g (125 mmol) AlCl₃ versetzt. Anschließend wurden rasch 11,9 g (52 mmol) 2-Bromisobuttersäurebromid zugegeben, 1 h bei 0°C und 2 h bei Raumtemperatur weiter gerührt. Die Mischung wurde noch 15 h unter Rückfluß erhitzt und dann analog Beispiel A aufgearbeitet. Das Rohprodukt wurde an 100 g Kieselgel (Hexan(Hexan/Methylenchlorid 1:1) chromatographiert. Die Ausbeute betrug 8,42 g (83 %).
¹H-NMR-Spektrum (100 MHz, CDCl₃): 7,7 (m), 7,2 (m), 3,35 (q), 2,70 (m), 2,58 (d), 1,95 (q), 1,25 (d), 0,93 (d).
Massenspektrum: 202 M⁺, korrektes Zerfallsmuster.

### Beispiel J

### 2-Methyl-6-isobutylinden (8)

Analog Beispiel D wurden 8,3 g (41 mmol) 2-Methyl-5-isobutyl-1-indanon (7) mit 2,4 g (62 mmol) NaBH₄ reduziert. Der nicht weiter gereinigte Alkohol wurde anschließend in Gegenwart von 0,4 g p-Toluolsulfonsäure in 100 ml Toluol bei 80°C weiter umgesetzt. Nach Chromatographie an 400 g Kieselgel (Hexan) erhielt man 7,17 g (95 %) 2-Methyl-6-isobutylinden (8).
¹H-NMR-Spektrum (100 MHz, CDCl₃): 7,1(m), 6,45 (m), 3,25 (m), 2,45 (d), 2,88 (q), 2,10 (d), 0,95 (d).
Massenspektrum: 184 M⁺, korrektes Zerfallsmuster.

### Beispiel K

### 2,5,6,7-Tetramethyl-1-indanon (9)

Eine Lösung von 6,01 g (50 mmol) 1,2,3-Trimethylbenzol in 30 ml Methylenchlorid p.a. wurde unter Eiskühlung mit 17,3 g (125 mmol) AlCl₃ versetzt. Anschließend wurden rasch 11,9 g (52 mmol) 2-Bromisobuttersäurebromid zugegeben, 1 h bei 0°C und 2 h bei Raumtemperatur weiter gerührt. Die Mischung wurde noch 15 h bei Raumtemperatur gehalten und dann analog Beispiel A aufgearbeitet. Das Rohprodukt wurde durch Destillation (0,05 Torr/96-107°C) gereinigt. Die Ausbeute betrug 8,1 g (86 %).
¹H-NMR-Spektrum (100 MHz, CDCl₃): 7,0 (m), 3,20 (q), 2,60 (m), 2,20 (m), 1,25 (d). Massenspektrum: 188 M⁺, korrektes Zerfallsmuster.

### Beispiel L

### 2,4,5,6-Tetramethylinden (10)

Analog Beispiel D wurden 1,50 g (8 mmol) 2,5,6,7-Tetramethyl-1-indanon (9) mit 0,45 g (12 mmol) NaBH₄ reduziert. Der nicht weiter gereinigte Alkohol wurde anschließend in Gegenwart von 0,1 g p-Toluolsulfonsäure in 100 ml Toluol weiter umgesetzt. Nach Chromatographie an 100 g Kieselgel (Hexan(Hexan/Methylenchlorid 9:1) erhielt man 0,88 g (65 %) 2,4,5,6-Tetramethylinden (10).
¹H-NMR-Spektrum (100 MHz, CDCl₃): 7,0 (s), 6,45 (m), 3,25 (m), 2,60 (m), 2,20 (m), 2,10 (d). Massenspektrum: 170 M⁺, korrektes Zerfallsmuster.

### Beispiel M

### Dimethylbis(2-Methyl-4,6-diisopropylindenyl)silan (11)

Eine Lösung von 4,9 g (22,8 mmol) der Isomerenmischung 4/4a in 25 ml THF wurde bei 0°C unter Ar-Schutz mit 9,2 ml (22,8 mmol) einer 2,5 M Butyllithiumlösung in Hexan versetzt und noch 1 h zum Rückfluß erhitzt. Die rote Lösung wurde anschließend bei Raumtemperatur zu einer Lösung von 1,5 g (11,4 ml) Dimethyldichlorsilan in 10 ml THF getropft und 8 h unter Rückfluß erhitzt. Der Ansatz wurde auf Eiswasser gegossen und ausgeethert. Die über Magnesiumsulfat getrocknete Etherphase wurde unter vermindertem Druck eingedampft. Das zurückgebliebene gelbliche Öl wurde anschließend an 500 g Kieselgel 60 chromatographiert. Mit einem Laufmittelgemisch aus Hexan/5 % Methylenchlorid konnten zunächst 1,4 g der Indenmischung 4/4a eluiert werden. Mit Hexan/8 % Methylenchlorid folgte das Ligandsystem 11. Das nach Abziehen des Laufmittels verbliebene viskose Öl konnte durch Verrühren mit Methanol im Eisbad kristallisiert werden. Es wurden 3,1 g eines gelblichen Feststoffs erhalten. Die Ausbeute betrug 56 % bzw. 84 % bezüglich umgesetztem Inden.
¹H-NMR-Spektrum (100 MHz, CDCl₃): Doppelbindungsisomere (3:1) 6,82-7,32 (m), 6,70 (m), 6,62 (m), 6,52 (m), 3,75 (s,br), 3,65 (s,br), 3,35 (s), 2,70-3,30 (m), 2,05-2,25 (m), 1,10-1,45 (m), 0,10-0,22 (m), -0,15 bis -0,32 (m).
Massenspektrum: 484 M⁺, korrekter Zerfall.

### Beispiel N

### Dimethylsilandiylbis(2-Methyl-4,6-diisopropylindenyl)zirkondichlorid (12)

Eine Lösung von 3,1 g (6,5 mmol) des Ligandsystems 11 in 25 ml Diethylether wurde bei Raumtemperatur unter Ar-Schutz mit 6,3 ml (16,2 mmol) einer 2,5 M Butyllithiumlösung in Hexan versetzt und über Nacht gerührt. Nach Zusatz von 10 ml Hexan wurde die beigefarbene Suspension filtriert und der Rückstand mit 20 ml Hexan gewaschen. Das Dilithiosalz wurde lange im Ölpumpenvakuum getrocknet und dann bei -78°C zu einer Suspension von 1,6 g (6,8 mmol) ZrCl₄ in 30 ml Methylenchlorid gegeben. Die Mischung wurde innerhalb 1 h auf Raumtemperatur erwärmt und noch 30 min bei dieser Temperatur gerührt. Nach dem Abziehen des Lösemittels wurde der orangebraune Rückstand mit 50 ml Hexan extrahiert. Nach Abziehen des Lösemittels erhielt man 2,6 g (60 %) des Komplexes 12 in Form eines gelben Pulvers. Das Verhältnis des Racemats zur meso-Form war 3:1. Durch Umkristallisation aus Hexan konnten 1,3 g (30 %) des Komplexes 12 als reines Racemat gewonnen werden (gelbes Kristallpulver).
¹H-NMR-Spektrum (100 MHz, CDCl₃): 7,27 (2,s,Arom.-H), 7,05 (2,s,Arom.-H), 6,80 (2,s,β-Ind-H), 2,6-3,2 (4,m,i-Pr-CH), 2,22 (6,s,Ind-CH₃), 1,15-1,40 (30,m,i-Pr-CH₃, Si-CH₃). Massenspektrum: 642 M⁺ (bzgl.⁹⁰Zr), korrektes Isotopenmuster, korrekter Zerfall.

### Beispiel O

### Dimethylbis(2,4,6-trimethylindenyl)silan (13)

Eine Lösung von 10,1 g (64 mmol) des Indens 2 in 50 ml THF wurde bei Raumtemperatur unter Ar-Schutz mit 25,5 ml (63,7 mmol) einer 2,5 M Butyllithiumlösung in Hexan versetzt und 1 h zum Rückfluß erhitzt. Die so erhaltene Lösung wurde bei Raumtemperatur zu einer Lösung von 4,1 g (32 mmol) Dimethyldichlorsilan in 20 ml THF getropft und 3 h zum Rückfluß erhitzt. Die Reaktionsmischung wurde auf Eiswasser gegossen und mehrmals ausgeethert. Die vereinigten organischen Phasen wurden über Natriumsulfat getrocknet und unter vermindertem Druck eingedampft. Der Rückstand wurde an 450 g Kieselgel 60 chromatographiert. Mit einem Laufmittelgemisch aus Hexan/5%Methylenchlorid ließen sich zunächst 2,5 g des Indens 2 eluieren. Mit Hexan/8%Methylenchlorid folgten 6,5 g des Ligandsystems 13 (Isomere). Die Ausbeute betrug 54 % bzw. 72 % bezüglich umgesetztem Inden 2.

### Beispiel P

### Dimethylsilandiylbis(2,4,6-trimethylindenyl)zirkondichlorid (14)

Eine Lösung von 2,0 g (5,4 mmol) des Ligandsystems 13 in 30 ml Diethylether wurde bei Raumtemeratur unter Ar-Schutz mit 6,6 ml (16,2 mmol) einer 2,5 M Butyllithiumlösung in Hexan versetzt und 5-6 h bei dieser Temperatur gerührt. Die Lösung wurde vollständig eingedampft. Der verbleibende feste Rückstand wurde portionsweise mit insgesamt 30 ml Hexan gewaschen und lange im Ölpumpenvakuum getrocknet. Das so erhaltene beigefarbene Pulver wurde bei -78°C zu einer Suspension von 1,23 g (5,5 mmol) Zirkontetrachlorid in 30 ml Methylenchlorid gegeben. Nach dem Aufwärmen auf Raumtemperatur wurde die Reaktionsmischung vollständig eingedampft und im Ölpumpenvakuum getrocknet. Der feste Rückstand bestand aus einer Mischung der racemischen mit der meso-Form im Verhältnis 1:1. Dieser wurde zunächst mit einer kleinen Menge Hexan gewaschen. Anschließend wurde mit insgesamt 120 ml Toluol extrahiert. Die Lösung wurde eingeengt und bei -35°C der Kristallisation überlassen. Es ließen sich 800 mg (28%) des Zirkonocens 14 als reines Racemat in Form orangefarbener Kristalle gewinnen.
¹H-NMR-Spektrum des Racemats (100 MHz, CDCl₃): 7,20 (s,2,Arom.-H), 6,97 (s,2,Arom.-H), 6,70 (s,2,β-Ind-H), 2,32 (s,6,CH₃), 2,27 (s,6,CH₃), 2,20 (s,6,CH₃), 1,27 (s,6,Si-CH₃).
Massenspektrum: 530 M⁺ (bzgl. ⁹⁰Zr), korrektes Isotopenmuster, korrekter Zerfall.

### Beispiel R

### Methylphenylbis(2-methyl-4,6-diisopropylindenyl)silan (15).

Eine Lösung von 10 g (46 mmol) des Indens 4/4a in 200 ml THF wurde unter Ar-Schutz bei Raumtemperatur mit 18,6 ml (46 mmol) einer 2,5 M Butyllithiumlösung in Hexan versetzt und 1 h zum Rückfluß erhitzt. Die Lösung wurde bei Raumtemperatur zu einer Lösung von 4,48 g (23 mmol) Methylphenyldichlorsilan in 30 ml THF getropft und 3 h zum Rückfluß erhitzt. Die Mischung wurde auf Eiswasser gegossen und mehrmals ausgeethert. Die vereinigten organischen Phasen wurden mit Natriumsulfat getrocknet und eingedampft. Der Rückstand wurde an 450 g Kieselgel 60 chromatographiert. Mit einem Laufmittelgemisch aus Hexan/Methylenchlorid (10:1) konnten zunächst 1,9 g nicht umgesetztes Inden 4/4a zurückgewonnen werden. Anschließend folgten 7,4 g des Ligandsystems 15 (Isomerengemisch). Die Ausbeute betrug 57 % bzw. 73 % bezüglich umgesetztem Inden.

### Beispiel S

### Methylphenylsilylbis(2-methyl-4,6-diisopropylindenyl)zirkondichlorid (16)

Eine Lösung von 7,4 g (13,5 mmol) des Ligandsystems 15 in 30 ml Diethylether wurde bei Raumtemperatur unter Ar-Schutz mit 11,2 ml (28 mmol) einer 2,5 M Butyllithiumlösung in Hexan versetzt und 16 h bei Raumtemperatur gerührt. Nach dem Abziehen des Lösemittels wurde der verbleibende Rückstand 3-4 h bei 40-50°C getrocknet und anschließend bei -78°C zu einer Suspension von 3,2 g (13,5 mmol) Zirkontetrachlorid in 40 ml Methylenchlorid gegeben. Nach dem Aufwärmen auf Raumtemperatur wurde das Lösemittel unter vermindertem Druck abgezogen. Der verbleibende feste Rückstand wurde im Ölpumpenvakuum getrocknet und mit 100 ml Hexan extrahiert. Nach Abziehen des Lösemittels erhält man 5,4 g (55%) des Zirkonocens 16 als Mischung der racemischen mit der meso-Form im Verhältnis 2:1 (orangebraunes Kristallpulver). Durch Umkristallisation aus Hexan ist die reine racemische Form erhältlich.
¹H-NMR-Spektrum der Isomerenmischung (100 MHz, CDCl₃): 6,6-8,2 (m,Arom.-H,β-Ind-H), 2,5-3,2 (m,i-Pr-H), 2,52 (s,CH₃), 2,32 (s,CH₃), 2,20 (s,CH₃), 1,90 (s,CH₃), 1,0-1,5 (m,i-Pr-CH₃,Si-CH₃).
Massenspektrum: 704 M⁺ (bzgl. ⁹⁰Zr), korrektes Isotopenmuster, korrekter Zerfall.

### Beispiel T

### 1,2-Bis(2-methyl-4,6-diisopropylindenyl)ethan (17)

Eine Lösung von 5,0 g (23,3 mmol) des Indens 4/4a in 50 ml THF wurde unter Ar-Schutz bei Raumtemperatur mit 18,6 ml (46 mmol) einer 2,5 M Butyllithiumlösung in Hexan versetzt und 1 h zum Rückfluß erhitzt. Die Lösung wurde bei -78°C zu einer Lösung von 2,18 g (11,0 mmol) 1,2-Dibromethan gegeben. Die Lösung wurde langsam auf Raumtemperatur erwärmt und über Nacht bei dieser Temperatur gerührt. Die Mischung wurde auf Eiswasser gegossen und mehrmals ausgeethert. Die vereinigten organischen Phasen wurden mit Natriumsulfat getrocknet und eingedampft. Der Rückstand wurde an 450 g Kieselgel 60 chromatographiert. Mit einem Laufmittelgemisch aus Hexan/Methylenchlorid (20:1 bis 10:1) konnten zunächst 1,2 g nicht umgesetztes Inden 4/4a zurückgewonnen werden. Anschließend folgten 1,7 g des Ligandsystems 17 (farbloser Feststoff). Die Ausbeute betrug 35 % bzw. 45 % bezüglich umgesetztem Inden.

### Beispiel U

### 1,2-Ethandiylbis(2-methyl-4,6-diisopropylindenyl)zirkondichlorid (18)

Eine Lösung von 1,6 g (3,52 mmol) des Ligandsystems 17 in 20 ml Diethylether wurde bei Raumtemperatur unter Ar-Schutz mit 3,5 ml (8,8 mmol) einer 2,5 M Butyllithiumlösung in Hexan versetzt und über Nacht gerührt. Der nach Abziehen des Lösemittels verbleibende Rückstand wurde mit Hexan gewaschen und lange im 8Ölpumpenvakuum getrocknet. Das so erhaltene Pulver wurde bei -78°C zu einer Suspension von 815 mg (3,5 mmol) Zirkontetrachlorid in 15 ml Methylenchlorid gegeben. Nach dem Erwärmen auf Raumtemperatur wurde noch 1 h gerührt und das Lösemittel unter vermindertem Druck entfernt. Der im Ölpumpenvakuum getrocknete Rückstand wurde mit Toluol extrahiert. Nach Abziehen des Lösemittels und Waschen mit Hexan erhielt man 1,5 g (70 %) des Zirkonocens 18 als Mischung der racemischen mit der meso-Form im Verhältnis 2:1 (orangefarbenes Pulver).

Durch Umkristallisation aus einem Toluol/Hexan-Gemisch ließen sich 700 mg (32 %) des reinen Racemats gewinnen.
¹H-NMR-Spektrum des Racemats (100 MHz, CDCl₃): 7,3 (s,Arom.-H), 7,0 (s,Arom.-H), 6,55 (s,β-Ind-H), 3,6 (s,C₂H₄), 2,6-3,2 (m,i-Pr-H), 2,2 (s,CH₃). Massenspektrum: 612 M⁺ (bzgl. ⁹⁰Zr), korrektes Isotopenmuster, korrekter Zerfall.

### Beispiel V

### 2-Methyl-6,7-benzoindan-1-on (19a) und 2-Methyl-4,5-benzoindan-1-on (19b)

Eine Lösung von 10 g (83 mmol) Naphthalin und 19 g (83 mmol) 2-Bromisobuttersäurebromid in 200 ml CH₂Cl₂ wurde bei Raumtemperatur innerhalb 30 min über einen Feststoffdosiertrichter mit 27,5 g (207 mmol) AlCl₃ versetzt. Nach 4 h wurde die Mischung analog Beispiel A aufgearbeitet. Das Rohprodukt wurde mit Essigester über eine kurze, mit Kieselgel gefüllte Säule filtriert. Nach dem Abziehen des Lösemittels erhielt man 15,5 g (95 %) der Isomerenmischung 19a/19b als gelbliches Öl. Das Isomerenverhältnis 19a:19b betrug 1:2.
¹H-NMR-Spektrum (100 MHz, CDCl₃): 19a: 9,15 (m,1H), 7,40-8,10 (m,5H), 3,47 (dd,1H), 2,62-2,95 (m,2H), 1,37 (d,3H); 19b: 7,4-8,0 (m,6H), 3,7 (dd,1H), 2,75-3,10 (m,2H), 1,40 (d,3H).
Massenspektrum: 196 M⁺, korrektes Zerfallsmuster.

### Beispiel W

### 2-Methyl-6,7-benzoindan-1-on (19a)

Es wurde die gleiche Ansatzgröße wie in Beispiel V gewählt. Das Naphthalin wurde zusammen mit dem AlCl₃ in CH₂Cl₂ vorgelegt und bei Raumtemperatur wurde langsam Bromisobuttersäurebromid zugetropft. Nach 1,5 h wurde die Mischung wie in Beispiel V aufgearbeitet. Nach Chromatographie an Kieselgel 60 mit einer Hexan/Essigester-Mischung erhielt man 11 g (67 %) des Indanons 19a.

### Beispiel X

### 2-Methyl-4,5-benzoinden (20a) und 2-Methyl-6,7-benzoinden (20b)

Eine Lösung von 7,8 g (39,7 mmol) der Isomerenmischung der Indanone 19a/19b (Beispiel V) in 400 ml einer THF/Methanol-Mischung (2:1) wurde bei Raumtemperatur portionsweise mit 2,2 g (59,5 mmol) Natriumborhydrid versetzt und 14 h gerührt. Die Lösung wurde auf mit HCI angesäuertes Eiswasser gegossen und ausgeethert. Die vereinigten organischen Phasen wurden mehrmals mit Wasser gewaschen und mit Natriumsulfat getrocknet. Das nach Abziehen des Lösemittels zurückgebliebene orangefarbene Öl wurde in 240 ml Toluol gelöst und mit 570 mg (3,15 mmol) p-Toluolsulfonsäure 15 min auf 80°C erhitzt. Die Lösung wurde bei Raumtemperatur mehrmals mit Wasser gewaschen, mit Natriumsulfat getrocknet und eingedampft. Der Rückstand wurde an 300 g Kieselgel 60 chromatographiert. Mit einem Laufmittelgemisch aus Hexan/Diisopropylether (20:1) konnten 4,7 g (65 %) der Isomerenmischung der Indene 20a/20b im Verhältnis 1:2 eluiert werden (farbloses Öl).
¹H-NMR-Spektrum (360 MHz,CDCl₃): Isomerenmischung 7,2-8,1 (m), 7,05 (m), 6,57 (m), 3,57 (s), 3,42 (m), 2,25 (d), 2,20 (d).
Molmasse: 180 M⁺, korrektes Zerfallsmuster.

### Beispiel Y

### Dimethylbis(2-methyl-4,5-benzoindenyl)silan (21)

Eine Lösung von 4,6 g (25,5 mmol) der Isomerenmischung der Indene 20a/20b (Beispiel Y) in 50 ml THF wurde bei Raumtemperatur mit 10,2 ml (25,5 mmol) einer 2,5 M Butyllithiumlösung in Hexan versetzt und 1 h zum Rückfluß erhitzt. Die rote Lösung wurde anschließend bei Raumtemperatur zu einer Lösung von 1,55 g (12 mmol) Dimethyldichlorsilan in 10 ml THF getropft und 5-6 h zum Rückfluß erhitzt. Die Reaktionslösung wurde auf Eiswasser gegossen und mehrmals ausgeethert. Die mit Natriumsulfat getrockneten vereinigten organischen Phasen wurden eingedampft und im Ölpumpenvakuum getrocknet. Der Rückstand wurde an 300 g Kieselgel 60 chromatographiert. Mit einem Laufmittelgemisch aus Hexan/3 % Essigester konnten zunächst 500 g nicht umgesetztes Edukt 20a/20b eluiert werden. Mit dem gleichen Laufmittel folgte anschließend das Ligandsystems 21. Dieses konnte nach Abziehen des Lösemittels durch Verrühren mit Hexan kristallisiert werden. Die Ausbeute betrug 1,7 g (34 % bezüglich Si bzw. 44 % bezüglich umgesetztes 20a/20b).
¹H-NMR-Spektrum (100 MHz, CDCl₃): Diastereomere (1:1) 7,2-8,2 (m), 4,05 (s), 2,45 (d), 2,35 (d), -0,22 (s), -0,32 (s), -0,35 (s).
Massenspektrum: 416 M⁺, korrektes Zerfallsmuster und Isotopenmuster.

### Beispiel AA

### 2-Methyl-α-acenaphthindan-1-on (23)

Eine Lösung von 20 g (129 mmol) α-Acenaphthen in 320 ml Methylenchlorid wurde bei Raumtemperatur mit 29,7 g (129 mmol) 2-Bromisobuttersäurebromid versetzt. Anschließend wurden innerhalb 15 min über einen Feststoffdosiertrichter 43,5 g (324 mmol) AlCl₃ zugegeben. Nach 30 min Rühren wurde auf Eiswasser gegossen und mit Methylenchlorid extrahiert. Die organische Phase wurde mit Wasser und einer NaHCO₃-Lösung gewaschen und mit NaSo₄ getrocknet. Der nach Abziehen des Lösemittels verbliebene Rückstand wurde über eine kurze Säule mit Kieselgel filtriert. Mit Hexan/Essigester (9:2) erhielt man 25 g (87 %) des Indanons 23.
¹H-NMR (CDCl₃, 100 MHz): 8,57 (d,1), 7,60 (t,1), 7,35 (d,1), 7,25 (s,1), 3,45 (q,1), 3,40 (s,4), 2,60-2,95 (m,2), 1,35 (d,3).

### Beispiel BB

### 2-Methyl-α-acenaphthinden (24)

Eine Lösung von 20 g (90 mmol) der Verbindung 23 in 250 ml einer THF/Methanol-Mischung (2:1) wurde zu einer Suspension von 3,8 g (100 mmol) NaBH₄ in 80 ml THF getropft. Die Mischung wurde 2 h bei Raumtemperatur gerührt und mit 100 ml Essigester und 100 ml halbkonz. HCI versetzt. Es wurde 10 min zum Rückfluß erhitzt und mit Essigester extrahiert. Die organische Phase wurde mit Wasser gewaschen und mit Na₂SO₄ getrocknet. Beim Einengen und Kühlen auf -35°C kristallisierten in mehreren Fraktionen insgesamt 16,3 g (88 %) der Verbindung 24.
¹H-NMR (CDCl₃, 100 MHz): 7,1-7,8 (m,4,Aromaten-H), 6,97 (m,1,Olefin-H), 3,37 (s,6,CH₂), 2,25 (d,3,CH₃).

### Beispiel CC

### Dimethylbis(2-methyl-α-acenaphthindenyl)silan (25)

10,8 g (52,4 mmol) der Verbindung 24 wurden analog Beispiel O deprotoniert und mit Dimethyldichlorsilan umgesetzt. Die organische Phase wurde eingedampft und der Rückstand wurde an Kieselgel chromatographiert. Mit Hexan/4%/Essigester konnten 6,2 g (51 %) des Ligandsystems 25 gewonnen werden.
¹H-NMR (CDCl₃, 100 MHz): Diastereomerenpaar 7,1-7,8 (m,Aromaten-H), 4,0(s,CH), 3,45(s,CH₂), 2,47(d,CH₃), 2,40(d,CH₃), -0,25(s,SiCH₃), -0,35(s,SiCH₃), -0,37(s,SiCH₃).

### Beispiel DD

### rac-Dimethylsilandiylbis(2-methyl-α-acenaphthindenyl)zirkondichlorid (26)

4,9 g (10,5 mmol) des Ligandsystems 25 wurden analog Beispiel P umgesetzt. Das Rohprodukt, bestehend aus der racemischen mit der meso-Form im Verhältnis 1:1 wurde aus Chloroform umkristallisiert. Man erhielt 1,3 g (20 %) des Racemats 26 in Form eines orangegelben Pulvers.
¹H-NNR (CDCl₃, 100 MHz): 7,0-7,8 (m,Aromaten-H), 3,1-3,4 (m,CH₂), 2,35 (s,CH₃), 1,35 (s,SiCH₃).

### Polymerisationsbeispiele:

### Beispiel 1

Ein trockener 24 dm³-Reaktor wurde mit Propylen gespült und mit 12 dm³ flüssigem Propylen befüllt. Dann wurden 35 cm³ toluolische Methylaluminoxanlösung (entsprechend 52 mmol Al, mittlerer Oligomerisierungsgrad p = 20) zugegeben und der Ansatz bei 30°C 15 Minuten gerührt.

Parallel dazu wurden 3,5 mg (0,005 mmol) rac-Dimethylsilyl (2-methyl-4,6-diisopropyl-1-indenyl)₂ zirkondichlorid in 13,5 cm³ toluolischer Methylaluminoxanlösung (20 mmol Al) gelöst und durch 15 minütiges Stehenlassen voraktiviert.

Die weinrote Lösung wurde dann in den Reaktor gegeben, durch Wärmezufuhr auf 75°c aufgeheizt (10°C/min) und das Polymerisationssystem 1 h durch Kühlung bei 70°C gehalten. Gestoppt wurde die Polymerisation durch Abgasen der überschüssigen Monomeren. Es wurden 2,11 kg Polypropylen erhalten.

Die Aktivität des Metallocens betrug somit 603 kg PP/g Metallocen x h.
VZ = 259 cm³/g, M_{w} = 305000 g/mol; M_{w}/Mₙ = 2,0; II = 96,0 %; SD = 400 g/dm³; MFI (230/5) = 8,5 dg/min.

### Vergleichsbeispiel 1

Beispiel 1 wurde mit dem Metallocen rac-Dimethylsilyl (2-methyl-1-indenyl)₂ zirkondichlorid wiederholt. Die Metallocenaktivität war 395 kg PP/g Metallocen x h.

VZ = 159 cm³/g, M_{w} = 158000 g/mol, M_{w}/Mₙ = 2,1 und der MFI (230/5) war 48 dg/min. Der Isotaktische Index (II) betrug 96,0 %.

### Beispiel 2

Beispiel 1 wurde mit dem Metallocen rac-Dimethylsilyl (2-methyl-4-isopropyl-1-indenyl)₂ zirkondichlorid wiederholt.

Die Metallocenaktivität war 460 kg PP/g Metallocen x h, VZ = 152 cm³/g, M_{w} = 147500 g/mol, M_{w}/Mₙ = 2,3 und MFI (230/5) = 51 dg/min.

### Vergleichsbeispiel 3

Beispiel 1 wurde mit rac-Dimethylsilyl-(1-indenyl)₂ zirkondichlorid wiederholt. Die Metallocenaktivität war 695 kg PP/g Metallocen x h.

VZ = 31 cm³/g, M_{w} = 18500 g/mol, M_{w}/Mₙ = 2,2, MFI (230/5) war nicht mehr meßbar.

### Vergleichsbeispiel 4

Beispiel 1 wurde mit dem Metallocen rac-Dimethylsilyl-(4,7-dimethyl-1-indenyl)₂ zirkondichlorid wiederholt. Die Metallocenaktivität war 195 kg PP/g Metalocen x h, VZ = 16 cm³/g, M_{w} = 9500 g/mol, M_{w}/Mₙ = 2,0, II = 87 %, der MFI (230/5) war nicht meßbar.

Die vier Vergleichsversuche zeigen, daß mit den am Indenylliganden in unterschiedlicher Weise substituierten Metallocenen bzw. mit dem unsubstituierten Metallocen hergestellte Polypropylene deutliche Molmassenunterschiede aufweisen. Unter Einbeziehung des erfindungsgemäßen Metallocens aus Beispiel 1 reicht die Spanne vom Wachsbereich (Vergleichsbeispiel 4) bis zum sehr hochmolekularen erfindungsgemäßen Polymer (Beispiel 1).

Diese Versuche belegen die Überlegenheit der in 2,4,6-Stellung substituierten Metallocene.

## Patentansprüche

1. Verfahren zur Herstellung einer am Fünfring und am Sechsring substituierten Verbindung der Formel IV oder deren Isomer der Formel IVa 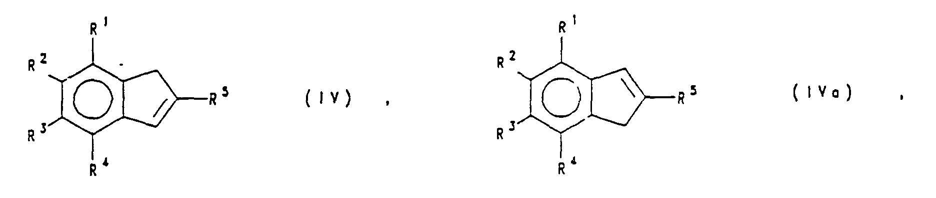 worin
R¹, R², R³, R⁴ und R⁵ gleich oder verschieden sind und Wasserstoff, (C₁-C₂₀)Alkyl, (C₆-C₁₄)Aryl, (C₁-C₁₀)Alkoxy, (C₂-C₁₀)Alkenyl, (C₇-C₂₀)Arylalkyl, (C₇-C₂₀)Alkylaryl, (C₆-C₁₀)Aryloxy, (C₁-C₁₀)Fluoralkyl, (C₆-C₁₀)Halogenaryl,
(C₂-C₁₀)Alkinyl, einen Rest -SiR⁶₃, wobei R⁶ für (C₁-C₁₀)Alkyl steht, ein Halogenatom oder einen heteroaromatischen Rest mit 5 oder 6 Ringgliedern, der ein oder mehrere Heteroatome enthalten kann, bedeuten, oder benachbarte Reste R¹-R⁴ bilden mit den sie verbindenden Atomen einen oder mehrere Ringe, **dadurch gekennzeichnet, daß** man eine Verbindung der Formel I 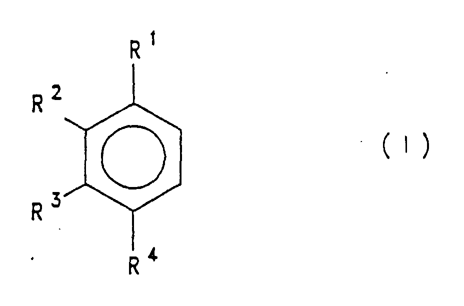 mit einer Verbindung der Formel II 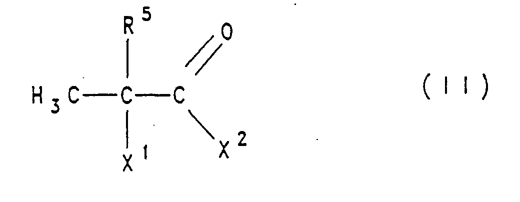 oder deren Anhydrid in Gegenwart eines Friedel-Crafts-Katalysators zu einer Verbindung der Formel III bzw. der Formel IIIa umsetzt, 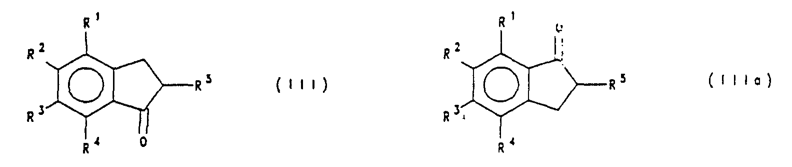 wobei R¹-R⁵ die genannten Bedeutungen besitzen und X¹ und X² gleich oder verschieden sind und für eine nudeophile Abgangsgruppe stehen, und diese nach bekannten Methoden durch Reduktion und Dehydratisierung in die Verbindung der Formel IV oder IV a überführt.

2. Verfahren gemäß Anspruch 1, **dadurch gekennzeichnet, daß** in den Formeln IV und IVa R¹, R², R³, R⁴ gleich oder verschieden sind und Wasserstoff, (C₁-C₁₀)Alkyl, (C₆-C₁₄)Aryl, (C₁-C₄)Alkoxy, (C₂-C₆)Alkenyl, (C₁-C₆)Fluoralkyl, ein Halogenatom oder einen heteroaromatischen Rest mit 5 oder 6 Ringgliedern, der ein oder mehrere Heteroatome enthalten kann, bedeuten, oder benachbarte Reste R¹-R⁴ bilden mit den sie verbindenden Atomen einen Ring, und R⁵ (C₁-C₁₀)Alkyl bedeutet.

3. Verfahren gemäß Anspruch 1 oder 2, **dadurch gekennzeichnet, daß** in den Formeln IV und IVa R¹, R², R³, R⁴ gleich oder verschieden sind und Wasserstoff oder (C₁-C₁₀)Alkyl bedeuten, oder die Reste R¹ und R² oder R³ und R⁴ bilden mit den sie verbindenden Atomen einen Ring, und R⁵ für Methyl steht

4. Verfahren gemäß einem oder mehreren der Ansprüche 1-3, **dadurch gekennzeichnet, daß** in Formel II X¹ und X² ein Halogenatom bedeuten.

5. Verfahren gemäß einem oder mehreren der Ansprüche 1-4, **dadurch gekennzeichnet, daß** als Friedel-Crafts-Katalysator AlCl₃ verwendet wird.

6. Verwendung einer Verbindung der Formel IV oder IVa gemäß einem oder mehreren der Ansprüche 1-3 als Zwischenprodukt bei der Herstellung von Metallocenkomplexen.

7. Verbindung der Formel VI mit am Fünfring und am Sechsring substituierten Indenylliganden 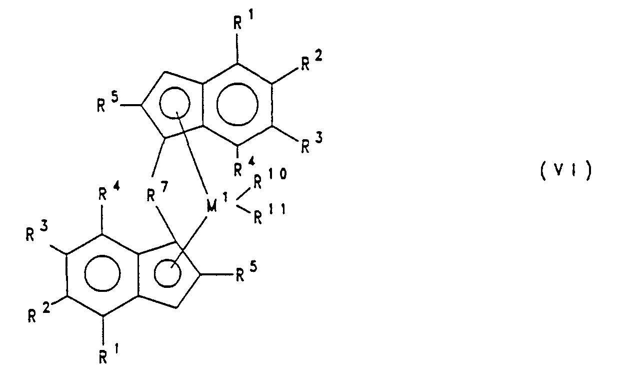 worin
M¹ Titan, Zirkon, Hafnium, Vanadium, Niob oder Tantal ist,
R¹, R², R³, R⁴ und R⁵ gleich oder verschieden sind und Wasserstoff, (C₁-C₂₀)Alkyl, (C₆-C₁₄)Aryl, (C₁-C₁₀)Alkoxy, (C₂-C₁₀)Alkenyl, (C₇-C₂₀)Arylalkyl, (C₇-C₂₀)Alkylaryl, (C₆-C₁₀)Aryloxy, (C₁-C₁₀)Fluoralkyl, (C₆-C₁₀)Halogenaryl, (C₂-C₁₀)Alkinyl, einen Rest -SiR⁶₃, wobei R⁶ für (C₁-C₁₀)Alkyl steht, ein Halogenatom oder einen heteroaromatischen Rest mit 5 oder 6 Ringgliedern, der ein oder mehrere Heteroatome enthalten kann, bedeuten, oder benachbarte Reste R¹-R⁴ bilden mit den sie verbindenden Atomen einen oder mehrere Ringe,
R⁷ ein Rest 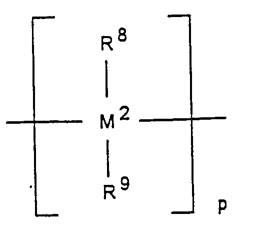 ist, wobei
M² Kohlenstoff, Silizium, Germanium oder Zinn bedeutet,
R⁸ und R⁹ gleich oder verschieden sind und Wasserstoff, (C₁-C₂₀)Alkyl, (C₆-C₁₄)Aryl, (C₁-C₁₀)Alkoxy, (C₂-C₁₀)Alkenyl, (C₇-C₂₀)Arylalkyl, (C₇-C₂₀)Alkylaryl, (C₆-C₁₀)Aryloxy, (C₁-C₁₀)Fluoralkyl, (C₆-C₁₀)Halogenaryl, (C₂-C₁₀)Alkinyl oder Halogen bedeuten, oder R⁸ und R⁹ zusammen mit dem sie verbindenden Atom einen Ring bilden, p 0 ist und R¹⁰ und R¹¹ gleich oder verschieden sind und Wasserstoff, (C₁-C₁₀)Alkyl, (C₁-C₁₀)Alkoxy, (C₆-C₁₀)Aryl, (C₆-C₁₀)Aryloxy, (C₂-C₁₀)Alkenyl, (C₇-C₄₀)Arylalkyl, (C₇-C₄₀)Alkylaryl, (C₈-C₄₀)Arylalkenyl, Hydroxy oder ein Halogenatom bedeuten.

8. Verbindung der Formel VI gemäß Anspruch 7, **dadurch gekennzeichnet, daß** M¹ Zirkon oder Hafnium, insbesondere Zirkon, ist,
R¹, R², R³, R⁴ gleich oder verschieden sind und Wasserstoff, (C₁-C₁₀)Alkyl, (C₆-C₁₄)Aryl, (C₁-C₄)Alkoxy, (C₂-C₆)Alkenyl, (C₁-C₆)Fluoralkyl, ein Halogenatom oder einen heteroaromatischen Rest mit 5 oder 6 Ringgliedern, der ein oder mehrere Heteroatome enthalten kann, bedeuten, oder benachbarte Reste R¹-R⁴ bilden mit den sie verbindenden Atomen einen Ring, und R⁵
(C₁-C₁₀)Alkyl bedeutet,
M² Kohlenstoff oder Silizium, insbesondere Silizium, bedeutet, R⁸ und R⁹ gleich oder verschieden sind und Wasserstoff, (C₁-C₆)Alkyl, (C₆-C₁₀)Aryl, (C₁-C₆)Alkoxy, (C₂-C₄)Alkenyl, (C₇-C₁₀)Arylalkyl, (C₇-C₁₀)Alkylaryl bedeuten oder R⁸ und R⁹ zusammen mit dem sie verbindenden Atom einen Ring bilden, p 1 oder 2, bevorzugt 1, ist, und R¹⁰ und R¹¹ gleich oder verschieden sind und Wasserstoff, (C₁-C₃)Alkyl, insbesondere Methyl, (C₁-C₃)Alkoxy, (C₆-C₈)Aryl, (C₆-C₈)Aryloxy, (C₂-C₄)Alkenyl, (C₇-C₁₀)Arylalkyl, (C₇-C₁₀)Alkylaryl, (C₈-C₁₂)Arylalkenyl oder ein Halogenatom, bevorzugt Chlor, bedeuten.

9. Verbindung der Formel VI gemäß Anspruch 7 oder 8, **dadurch gekennzeichnet, daß** M² Silizium ist und R⁸ und R⁹ gleich oder verschieden sind und (C₁-C₆)Alkyl oder (C₆-C₁₀)Aryl bedeuten.

10. Verbindung der Formel VI gemäß einem oder mehreren der Ansprüche 7-9, **dadurch gekennzeichnet, daß** die Indenylreste in Forme. Vl in 2,6-, 2,4,6-, 2,4,5-, 2,4,5,6- oder 2,4,5,6,7-Stellung substituiert sind.

11. Verbindung der Formel VI gemäß einem oder mehreren der Ansprüche 7-10, **dadurch gekennzeichnet, daß** die Indenylreste in Formel VI in 2,4,6- und 2,4,5- Stellung substituiert sind.

12. Verbindung der Formel VI gemäß Anspruch 10 oder 11, **dadurch gekennzeichnet, daß** die Indenylreste in Formel VI durch (C₁-C₄)Alkyl substituiert sind.

13. Verbindung einer Formel Vl gemäß Anspruch 10 oder 11, **dadurch gekennzeichnet, daß** die Indenylreste in Formel VI benzoannelliert sind.

14. Verwendung einer Verbindung der Formel VI gemäß einem oder mehreren der Ansprüche 7-13 als Katalysatorkomponente bei der Olefinpolymerisation.

15. Katalysator, enthaftend a) eine Verbindung der Formel VI gemäß einem oder mehreren der Ansprüche 7 - 13 und b) einen Cokatalysator.

16. Verfahren zur Herstellung eines Olefinpolmyers in Gegenwart eines Katalysators gemäß Anspruch 15.

17. Verbindung der Formel VI mit am Fünfring und am Sechsring substituierten Indenylliganden, die in 2,6-, 2,4,6-, 2,4,5,6- oder 2,4,5,6,7-Stellung substituiert sind, 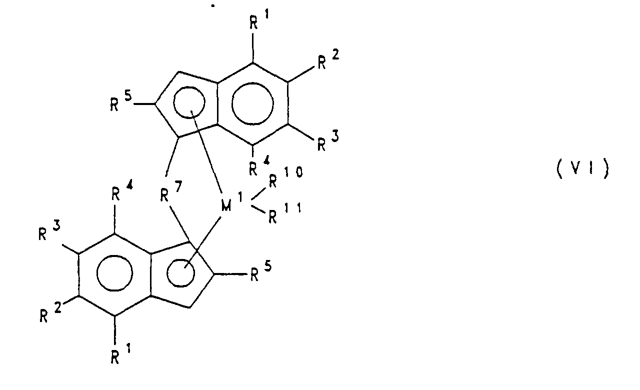 worin
M¹ Titan, Zirkon, Hafnium, Vanadium, Niob oder Tantal ist,
R¹, R², R³, R⁴ und R⁵ gleich oder verschieden sind und Wasserstoff, (C₁-C₂₀)Alkyl, (C₆-C₁₄)Aryl, (C₁-C₁₀)Alkoxy, (C₂-C₁₀)Alkenyl, (C₇-C₂₀)Arylalkyl, (C₇-C₂₀)Alkylaryl, (C₆-C₁₀)Aryloxy, (C₁-C₁₀)Fluoralkyl, (C₆-C₁₀)Halogenaryl, (C₂-C₁₀)Alkinyl, einen Rest -SiR⁶₃, wobei R⁶ für (C₁-C₁₀)Alkyl steht, ein Halogenatom oder einen heteroaromatischen Rest mit 5 oder 6 Ringgliedern, der ein oder mehrere Heteroatome enthalten kann, bedeuten, oder benachbarte Reste R¹-R⁴ bilden mit den sie verbindenden Atomen einen oder mehrere Ringe,
R⁷ ein Rest 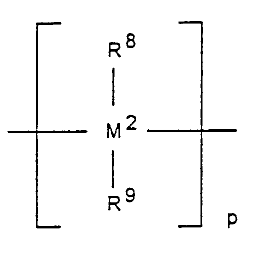 ist, wobei
M² Kohlenstoff, Silizium, Germanium oder Zinn bedeutet,
R⁸ und R⁹ gleich oder verschieden sind und Wasserstoff, (C₁-C₂₀)Alkyl, (C₆-C₁₄)Aryl, (C₁-C₁₀)Alkoxy, (C₂-C₁₀)Alkenyl, (C₇-C₂₀)Arylalkyl, (C₇-C₂₀)Alkylaryl, (C₆-C₁₀)Aryloxy, (C₁-C₁₀)Fluoralkyl, (C₆-C₁₀)Halogenaryl, (C₂-C₁₀)Alkinyl oder Halogen bedeuten, oder R⁸ und R⁹ zusammen mit dem sie verbindenden Atom einen Ring bilden, p 1, 2 oder 3 ist und
R¹⁰ und R¹¹ gleich oder verschieden sind und Wasserstoff, (C₁-C₁₀)Alkyl, (C₁-C₁₀)Alkoxy, (C₆-C₁₀)Aryl, (C₆-C₁₀)Aryloxy, (C₂-C₁₀)Alkenyl, (C₇-C₄₀)Arylalkyl, (C₇-C₄₀)Alkylaryl, (C₈-C₄₀)Arylalkenyl, Hydroxy oder ein Halogenatom bedeuten.

18. Verbindung der Formel VI gemäß Anspruch 17, **dadurch gekennzeichnet, daß** M¹ Zirkon oder Hafnium, insbesondere Zirkon, ist,
R¹, R², R³, R⁴ gleich oder verschieden sind und Wasserstoff, (C₁-C₁₀)Alkyl, (C₆-C₁₄)Aryl, (C₁-C₄)Alkoxy, (C₂-C₆)Alkenyl, (C₁-C₆)Fluoralkyl, ein Halogenatom oder einen heteroaromatischen Rest mit 5 oder 6 Ringgliedern, der ein oder mehrere Heteroatome enthalten kann, bedeuten, oder benachbarte Reste R¹-R⁴ bilden mit den sie verbindenden Atomen einen Ring, und R⁵
(C₁-C₁₀)Alkyl bedeutet,
M² Kohlenstoff oder Silizium, insbesondere Silizium, bedeutet, R⁸ und R⁹ gleich oder verschieden sind und Wasserstoff, (C₁-C₆)Alkyl, (C₆-C₁₀)Aryl, (C₁-C₆)Alkoxy, (C₂-C₄)Alkenyl, (C₇-C₁₀)Arylalkyl, (C₇-C₁₀)Alkylaryl bedeuten oder R⁸ und R⁹ zusammen mit dem sie verbindenden Atom einen Ring bilden, p 1 oder 2, bevorzugt 1, ist, und R¹⁰ und R¹¹ gleich oder verschieden sind und Wasserstoff, (C₁-C₃)Alkyl, insbesondere Methyl, (C₁-C₃)Alkoxy, (C₆-C₈)Aryl, (C₆-C₈)Aryloxy, (C₂-C₄)Alkenyl, (C₇-C₁₀)Arylalkyl, (C₇-C₁₀)Alkylaryl, (C₈-C₁₂)Arylalkenyl oder ein Halogenatom, bevorzugt Chlor, bedeuten.

19. Verbindung der Formel VI gemäß Anspruch 17 oder 18, **dadurch gekennzeichnet, daß** M² Silizium ist und R⁸ und R⁹ gleich oder verschieden sind und (C₁-C₆)Alkyl oder (C₆-C₁₀)Aryl bedeuten.

20. Verbindung der Formel VI gemäß einem oder mehreren der Ansprüche 17-19, **dadurch gekennzeichnet, daß** die Indenylreste in Formel VI in 2,4,6- Stellung substituiert sind.

21. Verbindung der Formel VI gemäß einem oder mehreren der Ansprüche 17 bis 20, **dadurch gekennzeichnet, daß** die Indenylreste in Formel VI durch (C₁-C₄)Alkyl substituiert sind.

22. Verwendung einer Verbindung der Formel VI gemäß einem oder mehreren der Ansprüche 17-21 als Katalysatorkomponente bei der Olefinpolymerisation.

23. Katalysator, enthaltend a) eine Verbindung der Formel VI gemäß einem oder mehreren der Ansprüche 17 - 21 und b) einen Cokatalysator.

24. Verfahren zur Herstellung eines Olefinpolmyers in Gegenwart eines Katalysators gemäß Anspruch 23.

25. Am Fünfring und am Sechsring substituierte Verbindung der Formel IV oder deren Isomer der Formel IVa 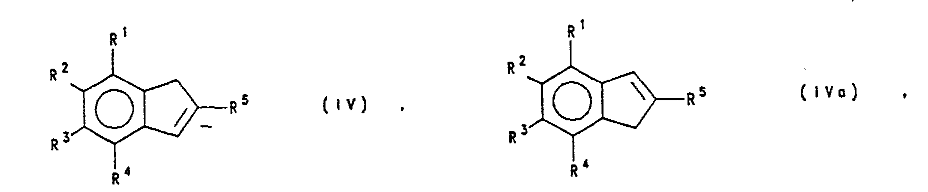 worin R¹, R², R³, R⁴ und R⁵ gleich oder verschieden sind und Wasserstoff, (C₁-C₂₀)Alkyl, (C₆-C₁₄)Aryl, (C₁-C₁₀)Alkoxy, (C₂-C₁₀)Alkenyl, (C₇-C₂₀)Arylalkyl, (C₇-C₂₀)Alkylaryl, (C₆-C₁₀)Aryloxy, (C₁-C₁₀)Fluoralkyl, (C₆-C₁₀)Halogenaryl, (C₂-C₁₀)Alkinyl, einen Rest -SiR⁶₃, wobei R⁶ für (C₁-C₁₀)Alkyl steht, ein Halogenatom oder einen heteroaraomatischen Rest mit 5 oder 6 Ringgliedern, der ein oder mehrere Heteroatome enthalten kann, bedeuten, oder benachbarte Reste R¹-R⁴ bilden mit den sie verbindenden Atomen einen oder mehrere Ringe, wobei a) R³ und R⁴ und R⁵ die genannte Bedeutung außer Wasserstoff haben und R¹ und R² Wasserstoff bedeuten, b) R³ und R⁵ die genannte Bedeutung außer Wasserstoff haben und R¹, R² und R⁴ Wasserstoff bedeuten, c) R² und R⁴ und R⁵ die genannte Bedeutung außer Wasserstoff haben und R¹ und R³ Wasserstoff bedeuten.

26. Am Fünfring und am Sechsring substituierte Verbindung der Formel IV oder deren Isomer der Formel IVa 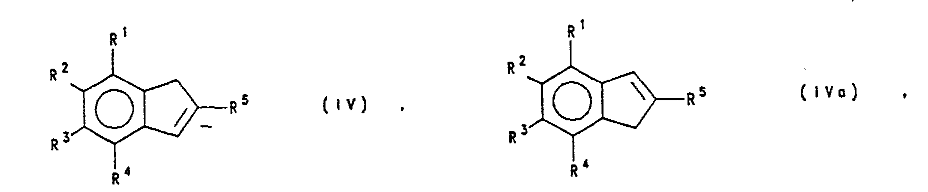 worin R¹, R², R³, R⁴ und R⁵ gleich oder verschieden sind und Wasserstoff, (C₁-C₂₀)Alkyl, (C₆-C₁₄)Aryl, (C₁-C₁₀)Alkoxy, (C₂-C₁₀)Alkenyl, (C₇-C₂₀)Arylalkyl, (C₇-C₂₀)Alkylaryl, (C₆-C₁₀)Aryloxy, (C₁-C₁₀)Fluoralkyl, (C₆-C₁₀)Halogenaryl, (C₂-C₁₀)Alkinyl, einen Rest -SiR⁶₃, wobei R⁶ für (C₁-C₁₀)Alkyl steht, ein Halogenatom oder einen heteroaraomatischen Rest mit 5 oder 6 Ringgliedern, der ein oder mehrere Heteroatome enthalten kann, bedeuten, oder benachbarte Reste R¹-R⁴ bilden mit den sie verbindenden Atomen einen oder mehrere Ringe, wobei R³ und R⁴ und R⁵ die genannte Bedeutung außer Wasserstoff haben und R¹ und R² Wasserstoff bedeuten und wobei R³ und R⁴ benzoanneliert sind.

27. 2-Methyl-6,7-benzoindan-1-on, 2-Methyl-4,5-benzoindan-1-on, 2-Methyl-5,7-diisopropyl-1-indanon, 2-Methyl-4,6-diisopropyl-1-indanon.

28. 2-Methyl-4,5-benzoinden, 2-Methyl-6,7-benzoinden.

## Claims

1. A process for the preparation of a compound of the formula IV substituted on the five- and on the six-membered ring or an isomer thereof of formula IVa 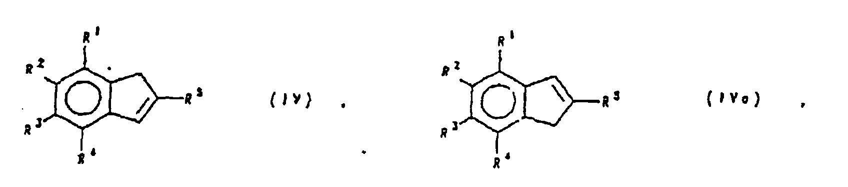 in which
R¹, R², R³, R⁴ and R⁵ are identical or different and are hydrogen, (C₁-C₂₀)alkyl, (C₆-C₁₄)aryl, (C₁-C₁₀)-alkoxy, (C₂-C₁₀) alkenyl, (C₇-C₂₀) arylalkyl, (C₇-C₂₀)-alkylaryl, (C₆-C₁₀)aryloxy, (C₁-C₁₀) fluoroalkyl, (C₆-C₁₀)halogenoaryl, (C₂-C₁₀) alkynyl, a radical -SiR⁶₃, in which R⁶ is (C₁-C₁₀)alkyl, a halogen atom or a heteroaromatic radical which has 5 or 6 ring members and can contain one or more hetero atoms, or adjacent radicals R¹-R⁴, with the atoms joining them, form one or more rings, which comprises reacting a compound of the formula I 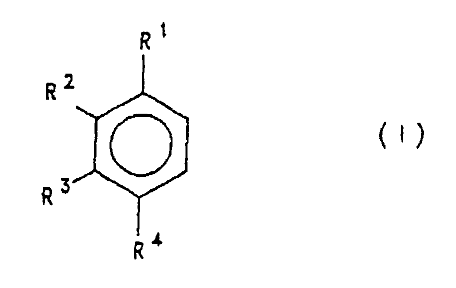 with a compound of the formula II 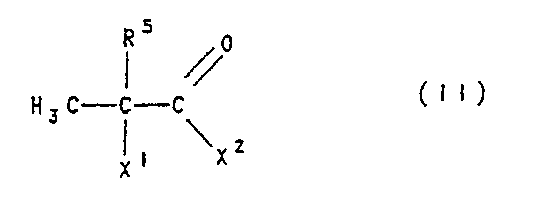 or an anhydride thereof, in the presence of a Friedel-Crafts catalyst to give a compound of the formula III or of the formula IIIa 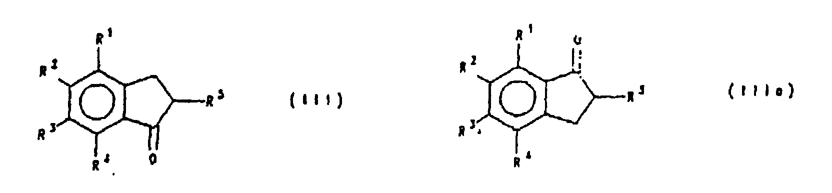 in which R¹-R⁵ have the meanings given and X¹ and X² are identical or different and are a nucleophilic leaving group, and converting this into the compound of the formula IV or IVa by reduction and dehydration by known methods.

2. The process as claimed in claim 1, wherein, in the formulae IV and IVa, R¹, R², R³ and R⁴ are identical or different and are hydrogen, (C₁-C₁₀)alkyl, (C₆-C₁₄)-aryl, (C₁-C₄)alkoxy, (C₂-C₆) alkenyl, (C₁-C₆) fluoroalkyl, a halogen atom or a heteroaromatic radical which has 5 or 6 ring members and can contain one or more hetero atoms, or adjacent radicals R¹-R⁴, with the atoms joining them, form a ring, and R⁵ is (C₁-C₁₀)alkyl.

3. The process as claimed in claim 1 or 2, wherein, in the formulae IV and IVa, R¹, R², R³ and R⁴ are identical or different and are hydrogen or (C₁-C₁₀)-alkyl, or the radicals R¹ and R² or R³ and R⁴, with the atoms joining them, form a ring, and R⁵ is methyl.

4. The process as claimed in one or more of claims 1-3, wherein, in formula II, X¹ and X² are a halogen atom.

5. The process as claimed in one or more of claims 1-4, wherein AlCl₃ is used as the Friedel-Crafts catalyst.

6. The use of a compound of the formula IV or IVa as claimed in one or more of claims 1-3 as an intermediate product in the preparation of a metallocene complex.

7. A compound of the formula VI having indenyl ligands substituted on the five- and on the six-membered ring 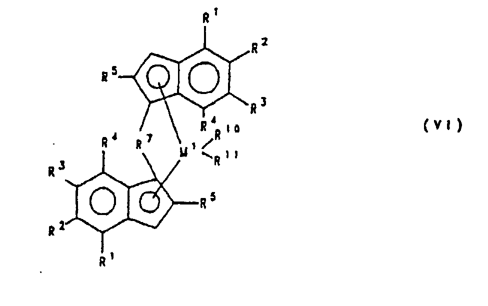 in which
M¹ is titanium, zirconium, hafnium, vanadium, niobium or tantalum,
R¹, R², R³, R⁴ and R⁵ are identical or different and are hydrogen, (C₁-C₂₀)alkyl, (C₆-C₁₄)aryl, (C₁-C₁₀)-alkoxy, (C₂-C₁₀) alkenyl, (C₇-C₂₀) arylalkyl, (C₇-C₂₀)-alkylaryl, (C₆-C₁₀)aryloxy, (C₁-C₁₀)fluoroalkyl, (C₆-C₁₀)halogenoaryl, (C₂-C₁₀) alkynyl, a radical -SiR⁶₃, in which R⁶ is (C₁-C₁₀)alkyl, a halogen atom or a heteroaromatic radical which has 5 or 6 ring members and can contain one or more hetero atoms, or adjacent radicals R¹-R⁴, with the atoms joining them, form one or more rings,
R⁷ is a radical 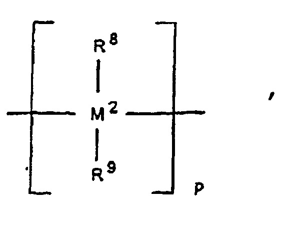 in which
M² is carbon, silicon, germanium or tin,
R⁸ and R⁹ are identical or different and are hydrogen, (C₁-C₂₀)alkyl, (C₆-C₁₄) aryl, (C₁-C₁₀) alkoxy, (C₂-C₁₀)alkenyl, (C₇-C₂₀) arylalkyl, (C₇-C₂₀)alkylaryl, (C₆-C₁₀)aryloxy, (C₁-C₁₀)fluoroalkyl, (C₆-C₁₀)halogenoaryl, (C₂-C₁₀)alkynyl or halogen, or
R⁸ and R⁹, together with the atom joining them, form a ring, p is 0 and
R¹⁰ and R¹¹ are identical or different and are hydrogen, (C₁-C₁₀)alkyl, (C₁-C₁₀)alkoxy, (C₆-C₁₀)aryl, (C₆-C₁₀)aryloxy, (C₂-C₁₀)alkenyl, (C₇-C₄₀)arylalkyl, (C₇-C₄₀) alkylaryl, (C₈-C₄₀) arylalkenyl, hydroxyl or a halogen atom.

8. A compound of the formula VI as claimed in claim 7, in which
M¹ is zirconium or hafnium, in particular zirconium,
R¹, R², R³ and R⁴ are identical or different and are hydrogen, (C₁-C₁₀)alkyl, (C₆-C₁₄)aryl, (C₁-C₄)alkoxy, (C₂-C₆) alkenyl, (C₁-C₆) fluoroalkyl, a halogen atom or a heteroaromatic radical which has 5 or 6 ring members and can contain one or more hetero atoms, or adjacent radicals R¹-R⁴, with the atoms joining them, form a ring, and R⁵ is (C₁-C₁₀)alkyl,
M² is carbon or silicon, in particular silicon, R⁸ and R⁹ are identical or different and are hydrogen, (C₁-C₆)alkyl, (C₆-C₁₀)aryl, (C₁-C₆)alkoxy, (C₂-C₄)-alkenyl, (C₇-C₁₀)arylalkyl or (C₇-C₁₀)alkylaryl, or R⁸ and R⁹, together with the atom joining them, form a ring, p is 1 or 2, preferably 1, and R¹⁰ and R¹¹ are identical or different and are hydrogen, (C₁-C₃)-alkyl, in particular methyl, (C₁-C₃)alkoxy, (C₆-C₈)-aryl, (C₆-C₈)aryloxy, (C₂-C₄) alkenyl, (C₇-C₁₀)-arylalkyl, (C₇-C₁₀) alkylaryl, (C₈-C₁₂) arylalkenyl or a halogen atom, preferably chlorine.

9. A compound of the formula VI as claimed in claim 7 or 8, in which M² is silicon and R⁸ and R⁹ are identical or different and are (C₁-C₆)alkyl or (C₆-C₁₀)aryl.

10. A compound of the formula VI as claimed in one or more of claims 7-9, in which the indenyl radicals in formula VI are substituted in the 2,6-, 2,4,6-, 2,4,5-, 2,4,5,6- or 2,4,5,6,7-position.

11. A compound of the formula VI as claimed in one or more of claims 7-10, in which the indenyl radicals in formula VI are substituted in the 2,4,6- and 2,4,5-position.

12. A compound of the formula VI as claimed in claim 10 or 11, in which the indenyl radicals in formula VI are substituted by (C₁-C₄)alkyl.

13. A compound of the formula VI as claimed in claim 10 or 11, in which the indenyl radicals in formula VI are benzo-fused.

14. The use of a compound of the formula VI as claimed in one or more of claims 7-13 as a catalyst component in olefin polymerization.

15. A catalyst comprising a) a compound of the formula VI as claimed in one or more of claims 7-13 and b) a cocatalyst.

16. A process for the preparation of an olefin polymer in the presence of a catalyst as claimed in claim 15.

17. A compound of the formula VI having indenyl ligands substituted on the five- and on the six-membered ring and in the 2,6-, 2,4,6-, 2,4,5,6- or 2,4,5,6,7-position 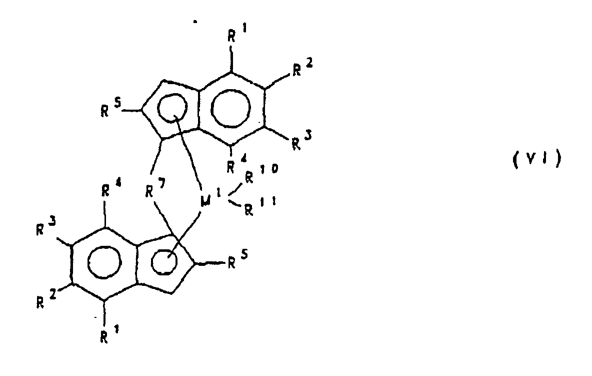 in which
M¹ is titanium, zirconium, hafnium, vanadium, niobium or tantalum,
R¹, R², R³, R⁴ and R⁵ are identical or different and are hydrogen, (C₁-C₂₀)alkyl, (C₆-C₁₄)aryl, (C₁-C₁₀)-alkoxy, (C₂-C₁₀) alkenyl, (C₇-C₂₀) arylalkyl, (C₇-C₂₀)-alkylaryl, (C₆-C₁₀)aryloxy, (C₁-C₁₀) fluoroalkyl, (C₆-C₁₀)halogenoaryl, (C₂-C₁₀)alkynyl, a radical -SiR⁶₃, in which R⁶ is (C₁-C₁₀)alkyl, a halogen atom or a heteroaromatic radical which has 5 or 6 ring members and can contain one or more hetero atoms, or adjacent radicals R¹-R⁴, with the atoms joining them, form one or more rings, R⁷ is a radical 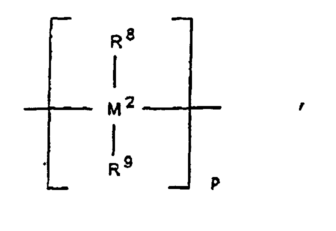 in which
M² is carbon, silicon, germanium or tin,
R⁸ and R⁹ are identical or different and are hydrogen, (C₁-C₂₀)alkyl, (C₆-C₁₄)aryl, (C₁-C₁₀)alkoxy, (C₂-C₁₀)alkenyl, (C₇-C₂₀)arylalkyl, (C₇-C₂₀)alkylaryl, (C₆-C₁₀)aryloxy, (C₁-C₁₀) fluoroalkyl, (C₆-C₁₀) halogenoaryl, (C₂-C₁₀)alkynyl or halogen, or
R⁸ and R⁹, together with the atom joining them, form a ring, p is 1,2 or 3 and
R¹⁰ and R¹¹ are identical or different and are hydrogen, (C₁-C₁₀)alkyl, (C₁-C₁₀)alkoxy, (C₆-C₁₀)aryl, (C₆-C₁₀)aryloxy, (C₂-C₁₀) alkenyl, (C₇-C₄₀)arylalkyl, (C₇-C₄₀) alkylaryl, (C₈-C₄₀) arylalkenyl, hydroxyl or a halogen atom.

18. The compound of the formula VI as claimed in claim 17, in which
M¹ is zirconium or hafnium, in particular zirconium,
R¹, R², R³ and R⁴ are identical or different and are hydrogen, (C₁-C₁₀)alkyl, (C₆-C₁₄)aryl, (C₁-C₄)alkoxy, (C₂-C₆)alkenyl, (C₁-C₆)fluoroalkyl, a halogen atom or a heteroaromatic radical which has 5 or 6 ring members and can contain one or more hetero atoms, or adjacent radicals R¹-R⁴, with the atoms joining them, form a ring, and R⁵ is (C₁-C₁₀)alkyl,
M² is carbon or silicon, in particular silicon, R⁸ and R⁹ are identical or different and are hydrogen, (C₁-C₆)alkyl, (C₆-C₁₀)aryl, (C₁-C₆)alkoxy, (C₂-C₄)-alkenyl, (C₇-C₁₀)arylalkyl or (C₇-C₁₀)alkylaryl, or R⁸ and R⁹, together with the atom joining them, form a ring, p is 1 or 2, preferably 1, and R¹⁰ and R¹¹ are identical or different and are hydrogen, (C₁-C₃)-alkyl, in particular methyl, (C₁-C₃)alkoxy, (C₆-C₈)-aryl, (C₆-C₈) aryloxy, (C₂-C₄) alkenyl, (C₇-C₁₀)-arylalkyl, (C₇-C₁₀) alkylaryl, (C₈-C₁₂) arylalkenyl or a halogen atom, preferably chlorine.

19. The compound of the formula VI as claimed in claim 17 or 18, in which M² is silicon and R⁸ and R⁹ are identical or different and are (C₁-C₆)alkyl or (C₆-C₁₀)aryl.

20. The compound of the formula VI as claimed in one or more of claims 17-19, in which the indenyl radicals in formula VI are substituted in the 2,4,6-position.

21. The compound of the formula VI as claimed in one or more of claims 17 to 20, in which the indenyl radicals in formula VI are substituted by (C₁-C₄)alkyl.

22. The use of a compound of the formula VI as claimed in one or more of claims 17-21 as a catalyst component in olefin polymerization.

23. A catalyst comprising a) a compound of the formula VI as claimed in one or more of claims 17-21 and b) a cocatalyst.

24. A process for the preparation of an olefin polymer in the presence of a catalyst as claimed in claim 23.

25. A compound of the formula IV substituted on the five- and on the six-membered ring or an isomer thereof of formula IVa 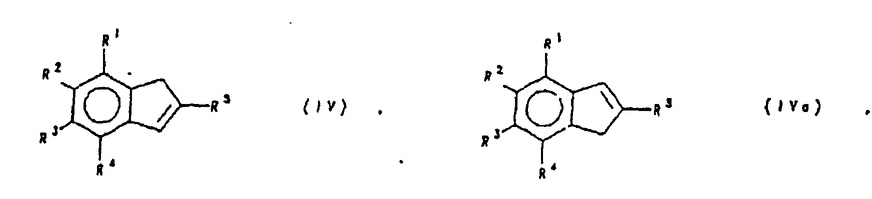 in which
R¹, R², R³, R⁴ and R⁵ are identical or different and are hydrogen, (C₁-C₂₀)alkyl, (C₆-C₁₄)aryl, (C₁-C₁₀)-alkoxy, (C₂-C₁₀)alkenyl, (C₇-C₂₀)arylalkyl, (C₇-C₂₀)-alkylaryl, (C₆-C₁₀)aryloxy, (C₁-C₁₀)fluoroalkyl, (C₆-C₁₀)halogenoaryl, (C₂-C₁₀)alkynyl, a radical -SiR⁶₃, in which R⁶ is (C₁-C₁₀)alkyl, a halogen atom or a heteroaromatic radical which has 5 or 6 ring members and can contain one or more hetero atoms, or adjacent radicals R¹-R⁴, with the atoms joining them, form one or more rings, where a) R³ and R⁴ and R⁵ have the above meaning, except hydrogen, and R¹ and R² are hydrogen, b) R³ and R⁵ have the above meaning, except hydrogen, and R¹, R² and R⁴ are hydrogen, c)
R² and R⁴ and R⁵ have the above meaning, except hydrogen, and R¹ and R³ are hydrogen.

26. A compound of the formula IV substituted on the five- and on the six-membered ring or an isomer thereof of formula IVa 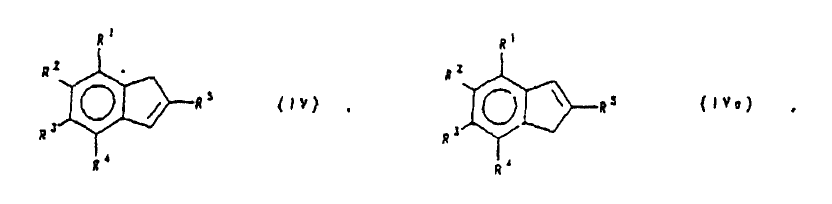 in which
R¹, R², R³, R⁴ and R⁵ are identical or different and are hydrogen, (C₁-C₂₀)alkyl, (C₆-C₁₄)aryl, (C₁-C₁₀)-alkoxy, (C₂-C₁₀)alkenyl, (C₇-C₂₀) arylalkyl, (C₇-C₂₀)-alkylaryl, (C₆-C₁₀)aryloxy, (C₁-C₁₀) fluoroalkyl, (C₆-C₁₀)halogenoaryl, (C₂-C₁₀)alkynyl, a radical -SiR⁶₃, in which R⁶ is (C₁-C₁₀)alkyl, a halogen atom or a heteroaromatic radical which has 5 or 6 ring members and can contain one or more hetero atoms, or adjacent radicals R¹-R⁴, with the atoms joining them, form one or more rings, where R³ and R⁴ and R⁵ have the above meaning, except hydrogen, and R¹ and R² are hydrogen, and where R³ and R⁴ are benzo-fused.

27. 2-Methyl-6,7-benzoindan-1-one, 2-methyl-4,5-benzoindan-1-one, 2-methyl-5,7-diisopropyl-1-indanone, 2-methyl-4,6-diisopropyl-1-indanone.

28. 2-Methyl-4,5-benzoindene, 2-methyl-6,7-benzoindene.

## Revendications

1. Procédé de préparation d'un composé substitué sur le cycle pentagonal ou sur le cycle hexagonal, de formule IV,
ou de son isomère de formule IVa 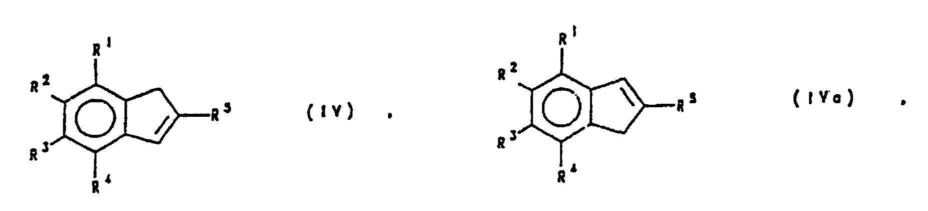 dans lesquelles
R¹, R², R³, R⁴ et R⁵ sont identiques ou différents et représentent un atome d'hydrogène, un groupe alkyle en C₁ à C₂₀, aryle en C₆ à C₁₄, alcoxy en C₁ à C₁₀, alcényle en C₂ à C₁₀, arylalkyle en C₇ à C₂₀, alkylaryle en C₇ à C₂₀, aryloxy en C₆ à C₁₀, fluoroalkyle en C₁ à C₁₀, halogénoaryle en C₆ à C₁₀, alcynyle en C₂ à C₁₀, un reste -SiR⁶₃ où R⁶ représente un groupe alkyle en C₁ à C₁₀, un atome d'halogène ou un reste hétéroaromatique présentant 5 ou 6 maillons de cycle et qui comprend éventuellement un ou plusieurs hétéroatomes, ou les restes R¹ à R⁴ qui sont voisins et les atomes qui les lient forment conjointement un ou plusieurs cycles, **caractérisé en ce que** l'on met à réagir un composé de formule I 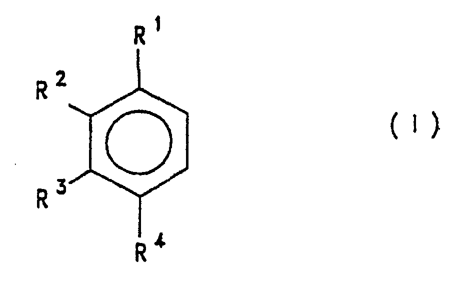 avec un composé de formule II 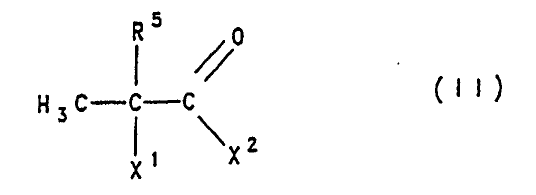 ou avec son anhydride, en présence d'un catalyseur de Friedel-Crafts, pour obtenir un composé de formule III ou de formule IIIa 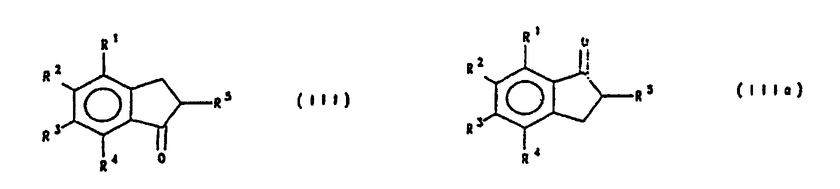 dans lesquelles R¹ à R⁵ prennent les significations susmentionnées, et X¹ et X² sont identiques ou différents et représentent un groupe quittant nucléophile, puis **en ce que** l'on transforme ces composés par une réduction et par une déshydratation, au moyen des procédés connus, en composés de formule IV ou IVa.

2. Procédé selon la revendication 1, **caractérisé en ce que** les restes R¹, R², R³ et R⁴ dans les formules IV et IVa sont identiques ou différents et représentent un atome d'hydrogène, un groupe alkyle en C₁ à C₁₀, aryle en C₆ à C₁₄, alcoxy en C₁ à C₄, alcényle en C₂ à C₆, fluoroalkyle en C₁ à C₆, un atome d'halogène ou un reste hétéroaromatique présentant 5 ou 6 maillons de cycle et qui comprend éventuellement un ou plusieurs hétéroatomes, ou les restes R¹ à R⁴ qui sont voisins et les atomes qui les lient forment conjointement un cycle, et R⁵ représente un groupe alkyle en C₁ à C₁₀.

3. Procédé selon la revendication 1 ou 2, **caractérisé en ce que** les restes R¹, R², R³ et R⁴ dans les formules IV et IVa sont identiques ou différents et représentent un atome d'hydrogène ou un groupe alkyle en C₁ à C₁₀, ou les restes R¹ et R², ou R³ et R⁴, et les atomes qui les lient forment conjointement un cycle, et R⁵ représente un groupe méthyle.

4. Procédé selon une ou plusieurs des revendications 1 à 3, **caractérisé en ce que** X¹ et X² dans la formule II représentent un atome d'halogéne.

5. Procédé selon une ou plusieurs des revendications 1 à 4, **caractérisé en ce que** l'on met en oeuvre, en tant que catalyseur de Friedel-Crafts, AlCl₃.

6. Mise en oeuvre d'un composé de formule IV ou IVa selon une ou plusieurs des revendications 1 à 3, en tant que produit intermédiaire pour la préparation de complexes métallocène.

7. Composé de formule VI présentant des ligands indényle substitués sur le cycle pentagonal ou sur le cycle hexagonal 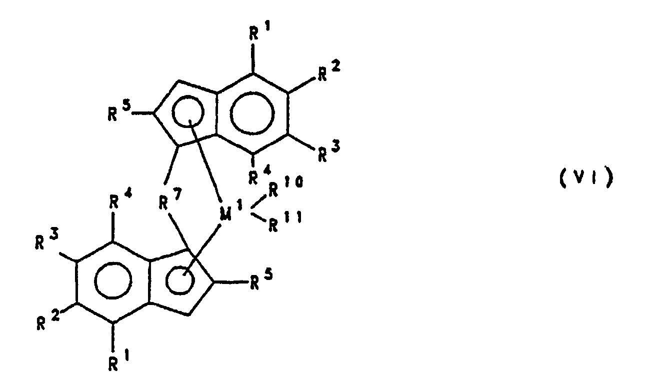 formule dans laquelle
M¹ représente un atome de titane, de zirconium, d'hafnium, de vanadium, de niobium ou de tantale,
R¹, R², R³, R⁴ et R⁵ sont identiques ou différents et représentent un atome d'hydrogène, un groupe alkyle en C₁ à C₂₀, aryle en C₆ à C₁₄, alcoxy en C₁ à C₁₀, alcényle en C₂ à C₁₀, arylalkyle en C₇ à C₂₀, alkylaryle en C₇ à C₂₀, aryloxy en C₆ à C₁₀, fluoroalkyle en C₁ à C₁₀, halogénoaryle en C₆ à C₁₀, alcynyle en C₂ à C₁₀, un reste -SiR⁶₃ où R⁶ représente un groupe alkyle en C₁ à C₁₀, un atome d'halogène ou un reste hétéroaromatique présentant 5 ou 6 maillons de cycle et qui comprend éventuellement un ou plusieurs hétéroatomes, ou les restes R¹ à R⁴ qui sont voisins et les atomes qui les lient forment conjointement un ou plusieurs cycles,
R7 représente un reste 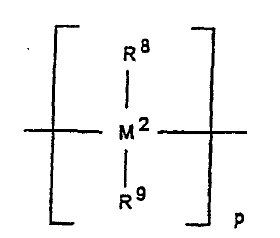 formule, dans laquelle
M² représente un atome de carbone, de silicium, de germanium ou d'étain,
R⁸ et R⁹ sont identiques ou différents et représentent un atome d'hydrogène, un groupe alkyle en C₁ à C₂₀, aryle en C₆ à C₁₄, alcoxy en C₁ à C₁₀, alcényle en C₂ à C₁₀, arylalkyle en C₇ à C₂₀, alkylaryle en C₇ à C₂₀, aryloxy en C₆ à C₁₀, fluoroalkyle en C₁ à C₁₀, halogénoaryle en C₆ à C₁₀, alcynyle en C₂ à C₁₀ ou un atome d'halogène, ou R⁸ et R⁹ et l'atome qui les lie forment conjointement un cycle, p vaut 0, et R¹⁰ et R¹¹ sont identiques ou différents et représentent un atome d'hydrogène, un groupe alkyle en C₁ à C₁₀, alcoxy en C₁ à C₁₀, aryle en C₆ à C₁₀, aryloxy en C₆ à C₁₀, alcényle en C₂ à C₁₀, arylalkyle en C₇ à C₄₀, alkylaryle en C₇ à C₄₀, arylalcényle en C₈ à C₄₀, hydroxyle ou un atome d'halogène.

8. Composé de formule VI selon la revendication 7, **caractérisé en ce que**
M¹ représente un atome de zirconium ou d'hafnium, en particulier un atome de zirconium,
R¹, R², R³ et R⁴ sont identiques ou différents et représentent un atome d'hydrogène, un groupe alkyle en C₁ à C₁₀, aryle en C₆ à C₁₄, alcoxy en C₁ à C₄, alcényle en C₂ à C₆, fluoroalkyle en C₁ à C₆, un atome d'halogène ou un reste hétéroaromatique présentant 5 ou 6 maillons de cycle et qui comprend éventuellement un ou plusieurs hétéroatomes, ou les restes R¹ à R⁴ qui sont voisins et les atomes qui les lient forment conjointement un cycle, et R⁵ représente un groupe alkyle en C₁ à C₁₀,
M² représente un atome de carbone ou de silicium, en particulier un atome de silicium, R⁸ et R⁹ sont identiques ou différents et représentent un atome d'hydrogène, un groupe alkyle en C₁ à C₆, aryle en C₆ à C₁₀, alcoxy en C₁ à C₆, alcényle en C₂ à C₄, arylalkyle en C₇ à C₁₀, alkylaryle en C₇ à C₁₀, ou R⁸ et R⁹ et l'atome qui les lie forment conjointement un cycle, p vaut 1 ou 2, de préférence 1, et
R¹⁰ et R¹¹ sont identiques ou différents et représentent un atome d'hydrogène, un groupe alkyle en C₁ à C₃, de préférence méthyle, un groupe alcoxy en C₁ à C₃, aryle en C₆ à C₈, aryloxy en C₆ à C₈, alcényle en C₂ à C₄, arylalkyle en C₇ à C₁₀, alkylaryle en C₇ à C₁₀, arylalcényle en C₈ à C₁₂ ou un atome d'halogène, de préférence un atome de chlore.

9. Composé de formule VI selon l'une quelconque des revendications 7 ou 8, **caractérisé en ce que** M² représente un atome de silicium et R⁸ et R⁹ sont identiques ou différents et représentent un groupe alkyle en C₁ à C₆ ou aryle en C₆ à C₁₀.

10. Composé de formule VI selon une ou plusieurs des revendications 7 à 9, **caractérisé en ce que** les restes indényle dans la formule VI sont substitués en position 2,6-, 2,4,6-, 2,4,5-, 2,4,5,6- ou 2,4,5,6,7-.

11. Composé de formule VI selon une ou plusieurs des revendications 7 à 10, **caractérisé en ce que** les restes indényle dans la formule VI sont substitués en position 2,4,6- et 2,4,5-.

12. Composé de formule VI selon la revendication 10 ou 11, **caractérisé en ce que** les restes indényle dans la formule VI sont substitués par un groupe alkyle en C₁ à C₄.

13. Composé de formule VI selon la revendication 10 ou 11, **caractérisé en ce que** les restes indényle dans la formule VI sont benzocondensés.

14. Mise en oeuvre du composé de formule VI selon une ou plusieurs des revendications 7 à 13 en tant que composant de catalyseur pour la polymérisation d'oléfines.

15. Catalyseur contenant a) un composé de formule VI selon une ou plusieurs des revendications 7 à 13 et b) un cocatalyseur.

16. Procédé de préparation d'un polymère d'oléfine en présence d'un catalyseur selon la revendication 15.

17. Composé de formule VI présentant des ligands indényle substitués, en position 2,6-, 2,4,6-, 2,4,5,6- ou 2,4,5,6,7-, sur le cycle pentagonal et sur le cycle hexagonal 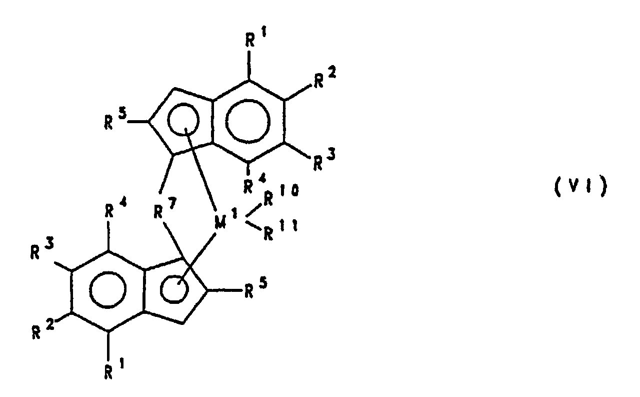 formule, dans laquelle
M¹ représente un atome de titane, de zirconium, d'hafnium, de vanadium, de niobium ou de tantale,
R¹, R², R³, R⁴ et R⁵ sont identiques ou différents et représentent un atome d'hydrogène, un groupe alkyle en C₁ à
C₂₀, aryle en C₆ à C₁₄, alcoxy en C₁ à C₁₀, alcényle en C₂ à C₁₀, arylalkyle en C₇ à C₂₀, alkylaryle en C₇ à C₂₀, aryloxy en C₆ à C₁₀, fluoroalkyle en C₁ à C₁₀, halogénoaryle en C₆ à C₁₀, alcynyle en C₂ à C₁₀, un reste -SiR⁶₃ où R⁶ représente un groupe alkyle en C₁ à C₁₀, un atome d'halogène ou un reste hétéroaromatique présentant 5 ou 6 maillons de cycle et qui comprend éventuellement un ou plusieurs hétéroatomes, ou les restes R¹ à R⁴ qui sont voisins et les atomes qui les lient forment conjointement un ou plusieurs cycles,
R⁷ représente un reste 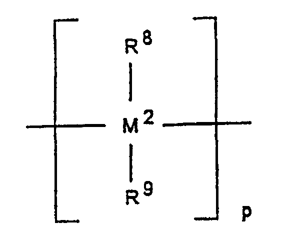 formule, dans laquelle
M² représente un atome de carbone, de silicium, de germanium ou d'étain,
R⁸ et R⁹ sont identiques ou différents et représentent un atome d'hydrogène, un groupe alkyle en C₁ à C₂₀, aryle en C₆ à C₁₄, alcoxy en C₁ à C₁₀, alcényle en C₂ à C₁₀, arylalkyle en C₇ à C₂₀, alkylaryle en C₇ à C₂₀, aryloxy en C₆ à C₁₀, fluoroalkyle en C₁ à C₁₀, halogénoaryle en C₆ à C₁₀, alcynyle en C₂ à C₁₀ ou un atome d'halogène, ou R⁸ et R⁹ et l'atome qui les lie forment conjointement un cycle, p vaut 1, 2 ou 3, et R¹⁰ et R¹¹ sont identiques ou différents et représentent un atome d'hydrogène, un groupe alkyle en C₁ à C₁₀, alcoxy en C₁ à C₁₀, aryle en C₆ à C₁₀, aryloxy en C₆ à C₁₀, alcényle en C₂ à C₁₀, arylalkyle en C₇ à C₄₀, alkylaryle en C₇ à C₄₀, arylalcényle en C₈ à C₄₀, hydroxyle ou un atome d'halogène.

18. Composé de formule VI selon la revendication 17, **caractérisé en ce que** M¹ représente un atome de zirconium ou d'hafnium, en particulier un atome de zirconium,
R¹, R², R³ et R⁴ sont identiques ou différents et représentent un atome d'hydrogène, un groupe alkyle en C₁ à C₁₀, aryle en C₆ à C₁₄, alcoxy en C₁ à C₄, alcényle en C₂ à C₆, fluoroalkyle en C₁ à C₆, un atome d'halogène ou un reste hétéroaromatique présentant 5 ou 6 maillons de cycle et qui comprend éventuellement un ou plusieurs hétéroatomes, ou les restes R¹ à R⁴ qui sont voisins et les atomes qui les lient forment conjointement un cycle, et R⁵ représente un groupe alkyle en C₁ à C₁₀,
M² représente un atome de carbone ou de silicium, en particulier un atome de silicium, R⁸ et R⁹ sont identiques ou différents et représentent un atome d'hydrogène, un groupe alkyle en C₁ à C₆, aryle en C₆ à C₁₀, alcoxy en C₁ à C₆, alcényle en C₂ à C₄, arylalkyle en C₇ à C₁₀, alkylaryle en C₇ à C₁₀, ou R⁸ et R⁹ et l'atome qui les lie forment conjointement un cycle, p vaut 1 ou 2, de préférence 1, et R¹⁰ et R¹¹ sont identiques ou différents et représentent un atome d'hydrogène, un groupe alkyle en C₁ à C₃, en particulier méthyle, un groupe alcoxy en C₁ à C₃, aryle en C₆ à C₈, aryloxy en C₆ à C₈, alcényle en C₂ à C₄, arylalkyle en C₇ à C₁₀, alkylaryle en C₇ à C₁₀, arylalcényle en C₈ à C₁₂ ou un atome d'halogène, de préférence un atome de chlore.

19. Composé de formule VI selon l'une des revendications 17 ou 18, **caractérisé en ce que** M² représente un atome de silicium et R⁸ et R⁹ sont identiques ou différents et représentent un groupe alkyle en C₁ à C₆ ou aryle en C₆ à C₁₀.

20. Composé de formule VI selon une ou plusieurs des revendications 17 à 19, **caractérisé en ce que** les restes indényle dans la formule VI sont substitués en position 2,4,6-.

21. Composé de formule VI selon une ou plusieurs des revendications 17 à 20, **caractérisé en ce que** les restes indényle dans la formule VI sont substitués par un groupe alkyle en C₁ à C₄.

22. Mise en oeuvre du composé de formule VI selon une ou plusieurs des revendications 17 à 21 en tant que composant de catalyseur pour la polymérisation d'oléfines.

23. Catalyseur contenant a) un composé de formule VI selon une ou plusieurs des revendications 17 à 21 et b) un cocatalyseur.

24. Procédé de préparation d'un polymère d'oléfine en présence d'un catalyseur selon la revendication 23.

25. Composé substitué sur le cycle pentagonal ou sur le cycle hexagonal et présentant la formule IV, ou son isomère de formule IVa 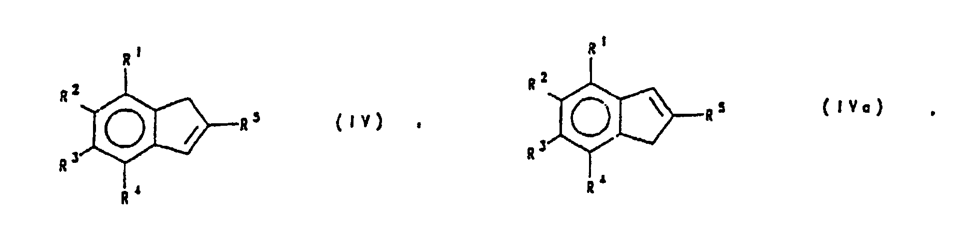 dans lesquelles R¹, R², R³, R⁴ et R⁵ sont identiques ou différents et représentent un atome d'hydrogène, un groupe alkyle en C₁ à C₂₀, aryle en C₆ à C₁₄, alcoxy en C₁ à C₁₀, alcényle en C₂ à C₁₀, arylalkyle en C₇ à C₂₀, alkylaryle en C₇ à C₂₀, aryloxy en C₆ à C₁₀, fluoroalkyle en C₁ à C₁₀, halogénoaryle en C₆ à C₁₀, alcynyle en C₂ à C₁₀, un reste - SiR⁶₃ où R⁶ représente un groupe alkyle en C₁ à C₁₀, un atome d'halogène ou un reste hétéroaromatique présentant 5 ou 6 maillons de cycle et qui comprend éventuellement un ou plusieurs hétéroatomes, ou les restes R¹ à R⁴ qui sont voisins et les atomes qui les lient forment conjointement un ou plusieurs cycles, a) R³ et R⁴ et R⁵ prenant la signification susmentionnée à l'exception de l'atome d'hydrogène, et R¹ et R² représentant un atome d'hydrogène, b) R³ et R⁵ prenant la signification susmentionnée à l'exception de l'atome d'hydrogène, et R¹, R² et R⁴ représentant un atome d'hydrogène, c) R² et R⁴ et R⁵ prenant la signification susmentionnée à l'exception de l'atome d'hydrogène, et R¹ et R³ représentant un atome d'hydrogène.

26. Composé substitué sur le cycle pentagonal ou sur le cycle hexagonal et présentant la formule IV, ou son isomère de formule IVa 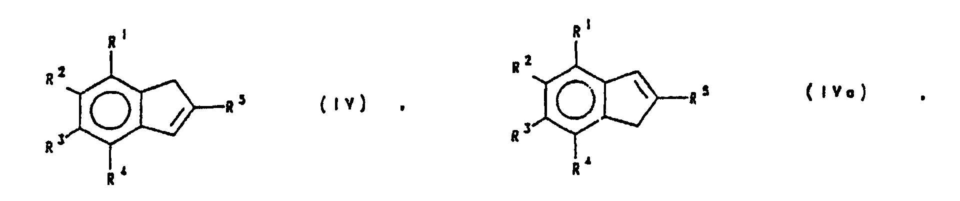 dans lesquelles R¹, R², R³, R⁴ et R⁵ sont identiques ou différents et représentent un atome d'hydrogène, un groupe alkyle en C₁ à C₂₀, aryle en C₆ à C₁₄, alcoxy en C₁ à C₁₀, alcényle en C₂ à C₁₀, arylalkyle en C₇ à C₂₀, alkylaryle en C₇ à C₂₀, aryloxy en C₆ à C₁₀, fluoroalkyle en C₁ à C₁₀, halogénoaryle en C₆ à C₁₀, alcynyle en C₂ à C₁₀, un reste - SiR⁶₃ où R⁶ représente un groupe alkyle en C₁ à C₁₀, un atome d'halogène ou un reste hétéroaromatique présentant 5 ou 6 maillons de cycle et qui comprend éventuellement un ou plusieurs hétéroatomes, ou les restes R¹ à R⁴ qui sont voisins et les atomes qui les lient forment conjointement un ou plusieurs cycles, R³ et R⁴ et R⁵ prenant la signification susmentionnée à l'exception de l'atome d'hydrogène, et R³ et R⁴ étant benzocondensés.

27. La 2-méthyl-6,7-benzoindan-1-one, la 2-méthyl-4,5-benzoindan-1-one, la 2-méthyl-5,7-diisopropyl-1-indanone, la 2-méthyl-4,6-diisopropyl-1-indanone.

28. Le 2-méthyl-4,5-benzoindène, le 2-méthyl-6,7-benzoindène.
